# EUROPEAN PATENT APPLICATION

(11) **EP 4 349 870 A1**
(43) Date of publication of application: **10.04.2024**
(21) Application number: 22815292.2
(22) Date of filing: 31.05.2022
(51) Int. Cl.: C07K 19/00, A61K 38/20, A61P 35/00, A61P 35/02, C12N 15/62, A61K 39/395

(54) **ANTI-CD3 ANTIBODY VARIANT, FUSION PROTEIN, AND APPLICATION**

(30) Priority: 02.06.2021 CN 202110628817
(71) Applicant: Qure Biotechnology (Shanghai) Co., Ltd., Shanghai 200120 (CN)
(72) Inventor: QU, Xiangdong, Shanghai 200120 (CN); PAN, Qin, Shanghai 200120 (CN)
(74) Representative: Cabinet Laurent & Charras
(86) International application number: PCT/CN2022/096501
(87) International publication number: WO 2022/253248

(57) **Abstract**

Provided are an anti-CD3 antibody variant, a fusion protein, and an application. An anti-CD3 antibody is mutated to obtain a variant thereof. The provided fusion protein comprises: (1) an anti-tumor-associated antigen (TAA)/anti-CD3 bispecific antibody; (2) anti-TAA, anti-CD3 and IL15/IL15Ra- -containing multifunctional fusion proteins; and (3) anti-TAA and IL15/IL15Ra-containing fusion proteins. Further provided are a nucleic acid molecule encoding an antibody molecule and a vector, and a pharmaceutical use of the antibody molecule.

## Description

### TECHNICAL FIELD

The present invention relates to the field of biomedicine technology, in particular to anti-CD3 antibody variant, fusion protein and application.

### BACKGROUND

T lymphocytes, abbreviated as T cells, derived from bone marrow lymphoid stem cells after differentiation and maturation in the thymus, are distributed to immune organs and tissues throughout the body through lymph and blood circulation to exert immune functions. T cells are efficient killer cells that can quickly eliminate virus infected cells and cancer cells. The killing effect of T cells requires the formation of immune synapses, which is a process highly dependent on TCR recognizing the complex formed by MHC molecules on the surface of antigen presenting cells and the presented antigen peptides. The activated immune synapses act a killing effect by releasing cytotoxin and cytokine. In the process of formation of immune synapses, due to the limitation of the distance between T cells and target cells, bispecific or multi-specific antibody directed T cells need to simulate the formation of immune synapses, by bridging one end to T cell TCR receptors by targeting CD3, while bridging the other end to target cells by targeting target cell surface antigens. CD3 molecule is expressed on the surface of all mature T cells, and can non-covalently bind to TCR, forming a complete TCR-CD3 complex that participates in the immune response to antigenic stimulus. It is currently the most widely and successfully used trigger molecule on the surface of immune effector cells in bispecific antibodies. The bispecific antibody targeting CD3 can bind to CD3 on the surface of T cells and tumor cell surface antigens respectively, thereby narrowing the distance between cytotoxic T cells (Tc or CTL) and tumor cells, and directly activating T cells, inducing them to directly kill cancer cells, without relying on traditional T cell double activation signals.

Among the disclosed CD3 antibodies, OKT3 is a milestone antibody, which pioneered monoclonal antibody therapy for treating diseases as the first therapeutic antibody to be launched. SP34 is currently one of the few disclosed CD3 antibodies that can have species specific reactions with monkeys. In recent years, there are new scientific breakthroughs in the field of bispecific antibodies, leading to the development of various types of new CD3 bispecific antibodies (CD3-BsAbs). Currently, over 100 kinds of CD3-BsAbs of different structures have been successfully developed. The numerous forms of antibodies determine many characteristics thereof, such as half-life period, immunogenicity, type of therapeutic response, ability to penetrate solid tumors, and so on. Blinatumomab (CD19xCD3 in BiTE form) from Amgen had FDA approval in 2014 and is the most successful CD3-BsAb bispecific antibody by far. This drug is used to treat patients with recurrent or refractory acute lymphoblastic leukemia (ALL). Compared with standard chemotherapy, more than 40% of patients receiving Blinatumomab treatment can achieve complete or partial response (CR/PR), and the median overall survival can be improved by several months. In addition to Blinatumomab, there are currently many other CD3-BsAbs undergoing clinical trials, such as CD19, CD20, CD38, BCMA, CD33, and CD123. Furthermore, in a phase 1/2 clinical trial, patients with acute myeloid leukemia (AML) were treated with Flotetuzumab (CD123xCD3-BsAb) from MacroGenics with an overall response rate (ORR) of 30%. In another phase 1/2 clinical trial of Epcoritamab (CD20xCD3-BsAb) from Abbvie/Gemab company, 44% of patients with diffuse lymphoid large B-cell tumor (DLBCL) showed complete response (CR) and 11% of that showed partial response (PR), while patients with follicular lymphoma had 100% response (PR). Many other CD20xCD3 bispecific antibodies have also achieved similar outstanding results. For example, in NSG mice, REGN1979 (CD20xCD3-BsAb) from Regeneron could have a better control over tumor growth than Rituximab (CD20 monoclonal antibody) from Roche, which further proved the efficacy of CD3 bispecific antibodies. The latest data on the CD20xCD3 T cell binding bispecific antibody Glofitamab (formerly known as CD20-TCB) from Roche treating the recurrent or refractory (R/R) diffuse large B-cell lymphoma (DLBCL) patients has been collected. This extended study involved DLBCL patients who were overtreated and highly refractory, with 58.3% of patients having no response to their initial treatment, and about one-third (33.1%) patients having received CAR-T cell therapy formerly. According to the Independent Review Committee (IRC) evaluation, at a median follow-up of 12.6 months, 39.4% of patients (61/155) achieved CR (the primary efficacy endpoint), and half of patients (51.6%; 80/155) achieved overall remission (the proportion of patients with partial response [PR]+complete response [CR]; the secondary efficacy endpoint). In terms of safety, cytokine release syndrome (CRS) was the most common adverse event, occurred in 63.0% of patients. CRS events are predictable, typically occur at a low level (mainly level 1 [47.4%] or level 2 [11.7%]) and occur at the initial dose, with only one patient discontinuing Glofitamab treatment due to CRS. The incidence of CRS at level 3 and above is relatively low (3.9%), and there are no level 5 events. Glofitamab has a novel "2:1" structural pattern, comprising two Fab regions targeting CD20 and one Fab region targeting CD3.

Amgen's Blinatumomab has shown in clinical trials that cytokine storm (CRS) is one of its most important toxic side effects. Tumor cells expressing CD19, when in the same environment as a large number of healthy B and T cells, may cause sudden CD3-BsAb mediated T cells activation, resulting in excessive release of inflammatory cytokines such as IFN-γ, IL-6 and TNF-α and so on, ultimately leading to symptoms from fever to multiple organ dysfunction. However, CRS is not a unique toxic side effect of Blinatumomab. In fact, CRS is frequently present in most CD3-BsAbs and CAR-T treatments. A humanized mouse model has shown that the main mediator of CD3-BsAb induced CRS is TNF-α produced by activated T cells, which leads to a large number of inflammatory cytokines secreted by monocytes. It has been found that CRS can be effectively alleviated by blocking upstream TNF-α and its downstream IL-1β or IL-6. In addition, several preclinical studies conducted in mouse and monkey models have shown that reducing the affinity of CD3 (i.e., using "weaker" CD3) can also reduce the level of cytokine storm.

Interleukin-15 (IL-15) is a 14-15 kDa cytokine that is crucial for the function of NK cells, NKT cells, and memory CD8+T cells. IL-15 binds to its receptor IL-15Rα to produce a complex with extremely high biological potency, IL-15 super agonist (IL-15 SA), which is then transduced and transported to target cells together. IL-15 SA strongly activates cells that express IL-15R, especially NK cells/T cells, thereby promoting anti-tumor and antiviral functions. IL-15 has a wide range of immune regulatory effects and can participate in regulating the survival, proliferation, and function of T cells, especially NK cells and memory CD8+T cells. IL-15 and IL-2 have very similar structures and belong to the spiral cytokine family. The heterotrimer receptor of IL-15 shares IL-2R/IL-15 Rβ (CD122) and the same γc chain (CD 132) with IL-2 receptor. Due to these common receptor components and the shared JAK 1/JAK 3/STAT 5 signaling pathway, IL-15 and IL-2 have the same functions comprising stimulating T cell proliferation, producing cytotoxic T lymphocytes, stimulating B cell to produce immunoglobulins, and producing and sustaining NK cells. In many adaptive immune responses, IL-2 and IL-15 have unique and often competitive roles. There are four main roles: 1. IL-2 can regulate the activation of Tregs cells, while IL-15 cannot. 2. IL-2 can inhibit T cell response by activating induced death of CD8+ effector T cells. 3. IL-15 plays an essential role in the differentiation of NK, CD8+ effector cells, and memory phenotype CD8+ T cells. 4. Preclinical studies have shown that the toxicity of IL-15 is different from that of IL-2, and IL-15 shows almost no vascular capillary leakage compared to IL-2. These factors make IL-15 a more promising cytokine in tumor immune strategies. At present, several IL-15 targeted products are still in clinical trials, with the most noteworthy being the IL-15 super agonist N-803, a fusion protein co-expressed by IL-15 protein N72D mutation combined with IL-15Ra and IgG1Fc. On May 23, 2022, ImmunityBio submitted the marketing application of IL-15 super agonist N-803 to FDA for combined treatment with Bacille Calmette-Guerin (BCG) for non-muscle invasive bladder cancer (NMIBC) that does not respond to BCG. The clinical data released in 2021 showed that the combined treatment of N-803+BCG had a significant effect, with a CR rate of 71% (59/83) and an average CR maintenance time of 24.1 months. A phase 1 clinical trial showed that the combined treatment of Nivolumab (PD-1 antibody, Opdivo) and N-803 for metastatic non-small cell lung cancer can significantly prolong the long-term survival of patients. Genentech collaborates with Xencor to develop IL-15 cytokine XmAb^{®}24306, and a clinical study is currently carried out for combined treatment of XmAb^{®}24306 and Tecentriq. SHR-1501 (IL-15 fusion protein) from Hengrui Pharmaceuticals was approved for clinical trials on May 14, 2019. Moreover, SHR-1316 (PD-L1 monoclonal antibody) will be used in combination with SHR-1501 for the treatment of advanced malignant tumor patients who have failed treatment.

At present, there is still in need of developing CD3 bispecific antibodies for treatment, and how to solve the challenges faced in solid tumors and improve the safety of CD3-BsAb bispecific antibodies require further research and development.

### DISCLOSURE OF THE INVENTION

The first purpose of the present invention is to provide an anti-CD3 antibody variant, which comprises a heavy chain variable region and a light chain variable region; the heavy chain variable region comprises: VHCDR1, VHCDR2, and VHCDR3; the light chain variable region comprises: VLCDR1, VLCDR2, and VLCDR3; the amino acid sequences of the anti-CD3 antibody comprises VHCDR1, VHCDR2, VHCDR3, VLCDR1, VLCDR2, and VLCDR3 shown in SEQ ID NO: 29-34; the mutation site of the anti-CD3 antibody variant comprises any one or more sites selected from the following group: position 10 of SEQ ID NO: 30, positions 2 and 7 of SEQ ID NO: 31, positions 3 and 4 of SEQ ID NO: 33, and positions 4, 5, 6, and 7 of SEQ ID NO: 34.

Preferably, the mutation is the substitution of amino acids.

Preferably, the mutation site of the variant comprises any one or more sites selected from the following group: A in position 10 substituted with E in SEQ ID NO: 30; G in position 2 substituted with S, and S in position 7 substituted with G in SEQ ID NO: 31; N in position 3 substituted with W, and K in position 4 substituted with L in SEQ ID NO: 33; Y in position 4 substituted with N or R, S in position 5 substituted with K, N in position 6 substituted with G, and L in position 7 substituted with G in SEQ ID NO: 34.

Preferably, the amino acid sequence of VHCDR2 is shown in SEQ ID NO: 35; alternatively, the amino acid sequence of VHCDR3 is shown in SEQ ID NO: 36; alternatively, the amino acid sequence of VLCDR2 is shown in SEQ ID NO: 37 or 38; alternatively, the amino acid sequence of VLCDR3 is shown in SEQ ID NO: 39, 40, or 41.

Preferably, the amino acid sequences of VHCDR1, VHCDR2, VHCDR3, VLCDR1, VLCDR2, and VLCDR3 are shown in any one of the following groups:
(1) SEQ ID NOs: 29, 35, 31, 32, 37, 39;
(2) SEQ ID NOs: 29, 35, 36, 32, 37, 40;
(3) SEQ ID NOs: 29, 35, 36, 32, 38, 41.

Preferably, the heavy chain variable region and/or light chain variable region of the antibody variant are selected from the heavy chain variable region and/or light chain variable region of the sequence as shown in or have at least 90% sequence identity with SEQ ID NO: 1, 2, or 3.

Preferably, there is a mutation used to form a disulfide bond between the heavy chain variable region and the light chain variable region, and the mutation site comprises any one or more of the following combination forms. The mutation site is EU numbering, and the heavy chain variable region is represented by VH, and the light chain variable region is represented by VL:

| | Disulfide bond mutation site | |
|---|---|---|
| | VH | VL |
| Combination 1 | 37C | 95C |
| Combination 2 | 44C | 100C |
| Combination 3 | 44C | 105C |
| Combination 4 | 45C | 87C |
| Combination 5 | 100C | 50C |
| Combination 6 | 100bC | 49C |
| Combination 7 | 98C | 46C |
| Combination 8 | 101C | 46C |
| Combination 9 | 105C | 43C |
| Combination 10 | 106C | 57C |

Preferably, the anti-CD3 antibody variant further comprises: a heavy chain constant region selected from human IgG1, IgG2, IgG3, or IgG4 or a variant thereof; and the light chain constant region selected from human κ, λ or a variant thereof; wherein the heavy chain constant region comprises: an Fc fragment or a variant thereof.

Preferably, the antibody variant is an scFv, which comprises a heavy chain variable region, a light chain variable region, and a linker connecting the heavy chain variable region and the light chain variable region. The amino acid sequence of the linker is preferably several GGGGS replicates, more preferably three GGGGS replicates.

Another purpose of the present invention is to provide the application of the anti-CD3 antibody variant in the preparation of drugs for inhibiting or treating cancer.

Preferably, the cancer is selected from the following cancers or occurs in the following areas: colorectal, breast, ovary, pancreas, stomach, prostate, kidney, cervix, bone marrow cancer, lymphatic cancer, leukemia, thyroid, endometrium, uterus, bladder, neuroendocrine, head and neck, liver, nasopharyngeal, testicle, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, and myelodysplastic syndrome.

Another purpose of the present invention is to provide a nucleic acid molecule that encodes the anti-CD3 antibody variant.

Another purpose of the present invention is to provide an expression vector comprising the aforementioned nucleic acid molecule.

Another purpose of the present invention is to provide a protein molecule against tumor associated antigen (TAA)/CD3, which is in a form of heterodimer, and comprises a first monomer and a second monomer;
The first monomer comprises: (a) an Fd fragment; (b) a light chain fragment and a first Fc chain; the second monomer comprises: (a) an Fd fragment; (b) a light chain fragment, an anti-CD3 antibody fragment, and a second Fc chain; the light chain fragment comprises a VL domain and a CL domain; the Fd fragment comprises a VH domain and a CH1 domain; in the first monomer or the second monomer, the light chain fragment pairs with the Fd fragment to form an anti-TAA Fab domain; wherein the light chain fragment of the first monomer is fused with the first Fc chain; the N-terminus of the anti-CD3 antibody fragment is fused with the light chain fragment of the second monomer, and the C-terminus is fused with the second Fc chain;
Alternatively, the first monomer comprises: (a) an Fd fragment; (b) a light chain fragment, a cytokine functional region, and a first Fc chain; the second monomer comprises: (a) an Fd fragment; (b) a light chain fragment, an anti-CD3 antibody fragment, and a second Fc chain; the cytokine functional region comprises IL-15 and IL-15Ra; wherein the N-terminus of the cytokine functional region is fused with the light chain fragment of the first monomer, and the C-terminus is fused with the first Fc chain; the N-terminus of the anti-CD3 antibody fragment is fused with the light chain fragment of the second monomer, and the C-terminus is fused with the second Fc chain;
Alternatively, the first monomer comprises: an anti-TAA antibody fragment, a cytokine functional region, and a first Fc chain; the second monomer comprises: an anti-TAA antibody fragment, an anti-CD3 antibody fragment, and a second Fc chain; wherein the N-terminus of the cytokine functional region is fused with the anti-TAA antibody fragment of the first monomer, and the C-terminus is fused with the first Fc chain; The N-terminus of the anti-CD3 antibody fragment is fused with the anti-TAA antibody fragment of the second monomer, and the C-terminus is fused with the second Fc chain;
The first Fc chain and the second Fc chain are interchangeable.

Preferably, the anti-TAA antibody fragment is in the form of scFv, comprising: a heavy chain variable region (VH), a light chain variable region (VL), and a linker connecting the heavy chain variable region and the light chain variable region; from the N-terminus to the C-terminus of the peptide chain, the fusion order of amino acids of the anti-TAA antibody fragment is "VH~VL", or "VL~VH"; wherein, "-" represents a linker; alternatively, the anti-CD3 antibody fragment is in the form of scFv, comprising: a heavy chain variable region (VH), a light chain variable region (VL), and a linker connecting the heavy chain variable region and the light chain variable region; from the N-terminus to the C-terminus of the peptide chain, the fusion order of the amino acids of the anti-CD3 antibody fragment is "VH~VL", or "VL~VH"; wherein, "-" represents a linker; alternatively, from the N-terminus to the C-terminus of the peptide chain, the fusion order of the amino acid fragments of the cytokine functional region is "IL-15~IL-15Ra", or "IL-15Ra~IL-15"; wherein, "-" represents a linker; preferably, the sequence of IL-15~IL-15Ra is shown in SEQ ID NO: 42, and the sequence of IL-15Ra~IL-15 is shown in SEQ ID NO: 43.

Preferably, the amino acid sequence of the anti-CD3 antibody fragment is selected from an antibody, an antibody fragment, a single domain antibody, or a humanized form thereof that specifically binds to CD3; preferably, the amino acid sequence of the anti-CD3 antibody fragment is selected from SP34, OKT3, UCTH1 or a derivative thereof.

Preferably, the anti-CD3 antibody fragment comprises a heavy chain variable region and a light chain variable region; the heavy chain variable region of the anti-CD3 antibody fragment comprises: VHCDR1, VHCDR2, and VHCDR3; the light chain variable region of the anti-CD3 antibody fragment comprises: VLCDR1, VLCDR2, VLCDR3; the amino acid sequences of VHCDR1, VHCDR2, VHCDR3, VLCDR1, VLCDR2, and VLCDR3 are shown in any set of sequences of the anti-CD3 antibody variant according to claim 5.

Preferably, IL-15Ra and IL-15 form an IL-15/IL-15Ra complex; IL-15 comprises: IL-15 and mutations, truncations, and various derivatives thereof that can bind to IL-15Ra; IL-15Ra comprises: IL-15Ra and mutations, truncations, and various derivatives thereof that can bind to IL-15; preferably, the IL-15/IL-15Ra complex includes but is not limited to any one of the mutation modes shown in the following combinations, and the numbering method is to start counting from the first amino acid in the amino acid sequence of IL-15 or IL-15Ra as the first position; preferably, the maternal sequence of IL-15 is shown in SEQ ID NO: 44; the maternal sequence of the IL-15Ra is shown in SEQ ID NO: 45:

| Combination | **IL15** | **IL15Ra** |
|---|---|---|
| 1 | wt | D96 |
| 2 | wt | D96/P97 |
| 3 | wt | D96/P97/A98 |
| 4 | E87C | D96/C97 |
| 5 | E87C | D96/P97/C98 |
| 6 | E87C | D96/C97/A98 |
| 7 | V49C | S40C |
| 8 | L52C | S40C |
| 9 | E89C | K34C |
| 10 | Q48C | G38C |
| 11 | E53C | L42C |
| 12 | C42S | A37C |
| 13 | L45C | G38C |
| 14 | L45C | A37C |

Preferably, the IL-15 includes but is not limited to any one of the mutation modes shown in the following combinations, and the numbering method is to start counting from the first amino acid in the amino acid sequence of IL-15 as the first position; preferably, the maternal sequence of IL-15 is shown in SEQ ID NO: 44:

| Combination | **IL15 mutation** |
|---|---|
| 1 | N1D |
| 2 | N4D |
| 3 | D8N |
| 4 | D30N |
| 5 | D61N |
| 6 | E64Q |
| 7 | N65D |
| 8 | Q108E |
| 9 | N1D/D61N |
| 10 | N1D/E64Q |
| 11 | N4D/D61N |
| 12 | N4D/E64Q |
| 13 | D8N/D61N |
| 14 | D8N/E64Q |
| 15 | D61N/E64Q |
| 16 | E64Q/Q108E |
| 17 | N1D/N4D/D8N |
| 18 | D61N/E64Q/N65D |
| 19 | N1D/D61N/E64Q/Q108E |
| 20 | N4D/D61N/E64Q/Q108E |

Preferably, the TAA is selected from: CD20, CD19, CD30, CD33, CD38, CD40, CD52, slamf7, GD2, CD24, CD47, CD133, CD239, CD276, PD-1, CEA, Epcam, Trop2, TAG72, MUC1, MUC16, mesothelin, folrl, CLDN18.2, PDL1, EGFR, EGFR VIII, C-MET, HER2, FGFR2, FGFR3, PSMA, PSCA, EphA2, ADAM17, 17-A1, NKG2D ligands, MCSP, LGR5, SSEA3, SLC34A2, BCMA, GPNMB or Glypican-3.

Preferably, the first Fc chain and the second Fc chain are polymerized to form an Fc fragment; the Fc fragment is selected from human IgG1 Fc, human IgG2 Fc, human IgG3 Fc, human IgG4 Fc or a variant thereof, preferably from IgG1 Fc, or human IgG4 Fc or a variant thereof; the protein molecule is in a form of Fc heterodimer; preferably, the Fc heterodimer includes but is not limited to a combination of the following mutations, according to EU numbering:

| Combina tion | FC | Heterodimer mutation (EU numbering) |
|---|---|---|
| 1 | first FC chain | T366Y |
| | second FC chain | Y407T |
| 2 | first FC chain | T366W |
| | second FC chain | T366S/L368A/Y407V |
| 3 | first FC chain | S354C/T366W |
| | second FC chain | Y349C/T366S/L368A/Y407V |
| 4 | first FC chain | S364H/F405A |
| | second FC chain | Y349T/T394F |
| 5 | first FC chain | T350V/L351Y/F405A/Y407V |
| | second FC chain | T350V/T366L/K392L/T394W |
| 6 | first FC chain | K392D/K409D |
| | second FC chain | E356K/D399K |
| 7 | first FC chain | D221EP228E/L368E |
| | second FC chain | D221R/P228R/K409R |
| 8 | first FC chain | K360EK409W |
| | second FC chain | Q347R/D399V/F405T |
| 9 | first FC chain | K360E/K409W/Y349C |
| | second FC chain | Q347R/D399V/F405T/S354C |
| 10 | first FC chain | K370E/K409W |
| | second FC chain | E357ND399VF405T |
| 11 | first FC chain | F405L |
| | second FC chain | K409R |
| 12 | first FC chain | K360D/D399M/Y407A |
| | second FC chain | E345R/Q347R/T366V/K409V |
| 13 | first FC chain | Y349S/K370Y/T366M/K409V |
| | second FC chain | E356G/E357D/S364Q/Y407A |
| 14 | first FC chain | L351D/L368E |
| | second FC chain | L351K/T366K |
| 15 | first FC chain | GQPFRPEVHLIPPSREMTKNQVSLTCLA RGFYPKDAVEWESNGQPENNYKTTPS RQEPSQGTTTFAVTSKLTVDKSRWQQ GNVFSCSVMHEALHNHYTQKTEL |
| | second FC chain | GQPREPQVYTPPPSEEAINELVILTCLV KGFYPSDAVEWLQGSQELPREKYLTW APVLDSDGSFFLYSLRVAAEDWKKGD TFSCSVMHEALHNHYTQKSDR |
| 16 | first FC chain | L368D/K370S |
| | second FC chain | E357Q/S364K |
| 17 | first FC chain | **S354C/T366W/K409A** |
| | second FC chain | Y349C/T366S/L368A/Y407V/**F405K** |
| 18 | first FC chain | S354C/T366W/**F405K/K360E/Q347E** |
| | second FC chain | Y349C/T366S/L368A/Y407V/**Q347R/T394 W** |
| 19 | first FC chain | T366W/**K409A** |
| | second FC chain | T366S/**L368G/Y407A/F405K** |
| 20 | first FC chain | knobs (**T366W/F405K**) |
| | second FC chain | holes (T366S/**L368G/Y407A/K409A**) |
| 21 | first FC chain | Q347A/S364K/T366VK370T/K392YF405S /Y407V/K409W/T41 1N |
| | second FC chain | Q347E/Y349AL351F/S364T/T366V//K370 T/T394D/V397L/D399E/ D401Q/F405A/Y407S/K409R/T411R |
| 22 | first FC chain | K274Q/N276K/Y300F/A339T/Q347A/S364 K/T366V/K370T/**N384S** /K392Y/**V397M**/F405S/Y407V/K409W/T4 11N/**V422I/H435R/Y436F** |
| | second FC chain | Q347E/Y349A/L351F/S364T/T366V/K370 T/T394D/V397L/D399E/ D401Q/F405A/Y407S/K409R/T411R |

Preferably, the protein molecule comprises an Fc fragment that selectively eliminates immune effector functions, including but not limited to a combination of the following mutations, according to EU numbering:

| **IgG** | FC Mutate(EU numbing) |
|---|---|
| **IgG1** | L234A,L235A |
| | L234A,L235A,P329G |
| | L234F,L235E,P331S |
| | D265A,N297A |
| | L234F,L235E,N297A |
| | L234F,L235E,D265A |
| | L234A,L235E,P331S |
| | L234A,L235E,N297A |
| | L234A,L235E,D265A |
| | L234AL235AP331S |
| | L234A,L235A,N297A |
| | L234A,L235A,D265A |
| | L235E,D265A,P331S |
| | L235E,N297A,P331S |
| | L235E,N297A |
| | L235AD265AP331S |
| | L235A,N297A,P331S |
| | N297Q |
| | N297A |
| | N297G |
| | A287C, N297G, L306C |
| | R292C, N297G, V302C |
| **hIgG4** | S228P,L235E,P329G |
| | S228P,L235E |
| | S228P,F234A,L235E |
| | S228P,F234A,L235A |
| **IgG2m4** | ASTKGPSVFPLAPCSRSTSESTAALGCLVKDYFPEPVTVSWNSGALT SGVHTFPAVLQSSGLYSLSSVVTVPSSNFGTQTYTCNVDHKPSNTKV DKTVERKCCVECPPCPAPPVAGPSVFLFPPKPKDTLMISRTPEVTCVV VDVSQEDPEVQFNWYVDGVEVHNAKTKPREEQFNSTYRVVSVLTV LHQDWLNGKEYKCKVSNKGLPSSIEKTISKAKGQPREPQVYTLPPSQ EEMTKNQVSLTCLVKGFYPSDIAVEWESNGQPENNYKTTPPVLDSD GSFFLYSRLTVDKSRWQEGNVFSCSVMHEALHNHYTQKSLSLSLGK |
| | N297A |
| | N297Q |
| | N297G |

Preferably, the anti-tumor associated antigen (TAA)/anti-CD3 protein molecule is obtained by fusing amino acid fragments shown in any one of the following groups of sequences:
(1) SEQ ID NO:05, SEQ ID NO:06, SEQ ID NO:07;
(2) SEQ ID NO:08, SEQ ID NO:06, SEQ ID NO:07;
(3) SEQ ID NO:09, SEQ ID NO:06, SEQ ID NO:07;
(4) SEQ ID NO:10, SEQ ID NO:06, SEQ ID NO:07;
(5) SEQ ID NO:05, SEQ ID NO:17, SEQ ID NO:07;
(6) SEQ ID NO:01, SEQ ID NO:18, SEQ ID NO:07;
(7) SEQ ID NO:19, SEQ ID NO:17, SEQ ID NO:07;
(8) SEQ ID NO:19, SEQ ID NO:18, SEQ ID NO:07;
(9) SEQ ID NO:20, SEQ ID NO:21;
(10) SEQ ID NO:22, SEQ ID NO:21;
(11) SEQ ID NO:20, SEQ ID NO:23;
(12) SEQ ID NO:22, SEQ ID NO:23;
(13) SEQ ID NO:24, SEQ ID NO:25;
(14) SEQ ID NO:26, SEQ ID NO:25;
(15) SEQ ID NO:24, SEQ ID NO:27;
(16) SEQ ID NO:26, SEQ ID NO:27.

Another purpose of the present invention is to provide a nucleic acid molecule that encodes the anti-tumor associated antigen (TAA)/anti-CD3 protein molecule.

Another purpose of the present invention is to provide an application of the anti-tumor associated antigen (TAA)/anti-CD3 protein molecule in the preparation of drugs for inhibiting or treating cancer, wherein the cancer is selected from the following cancers or occurs in the following areas: colorectal, breast, ovary, pancreas, stomach, prostate, kidney, cervix, bone marrow cancer, lymphatic cancer, leukemia, thyroid, endometrium, uterus, bladder, neuroendocrine, head and neck, liver, nasopharyngeal, testicle, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, and myelodysplastic syndrome.

Another purpose of the present invention is to provide an anti-tumor associated antigen (TAA) and IL-15/IL-15Ra-containing fusion protein, comprising two polypeptides, any one of which comprises: an anti-TAA antibody fragment, a cytokine functional region, and an Fc fragment; the anti-TAA antibody fragment is in the form of scFv; the cytokine functional region comprises IL-15 and IL-15Ra; the N-terminus of the cytokine functional region is fused with the anti-TAA antibody fragment, and the C-terminus is fused with the Fc fragment.

Preferably, in the anti-tumor associated antigen (TAA) and IL-15/IL-15Ra-containing fusion protein, the IL-15Ra and IL-15 form an IL-15/IL-15Ra complex; the IL-15 comprises: IL-15 and mutations, truncations, and various derivatives thereof that can bind to IL-15Ra; the IL-15Ra comprises: IL-15Ra and mutations, truncations, and various derivatives thereof that can bind to IL-15; preferably, the IL-15/IL-15Ra complex includes but is not limited to any one of the mutation modes shown in the following combinations, wherein the numbering method is to start counting from the first amino acid in the amino acid sequence of IL-15 or IL-15Ra as the first position; preferably, the maternal sequence of IL-15 is shown in SEQ ID NO: 44; the maternal sequence of IL-15Ra is shown in SEQ ID NO: 45:

| Combination | **IL15** | **IL15Ra** |
|---|---|---|
| 1 | wt | D96 |
| 2 | wt | D96/P97 |
| 3 | wt | D96/P97/A98 |
| 4 | E87C | D96/C97 |
| 5 | E87C | D96/P97/C98 |
| 6 | E87C | D96/C97/A98 |
| 7 | V49C | S40C |
| 8 | L52C | S40C |
| 9 | E89C | K34C |
| 10 | Q48C | G38C |
| 11 | E53C | L42C |
| 12 | C42S | A37C |
| 13 | L45C | G38C |
| 14 | L45C | A37C |

Preferably, in the anti-tumor associated antigen (TAA) and IL-15/IL-15Ra-containing fusion protein, the IL-15 includes but is not limited to any one of the mutation modes shown in the following combinations, and the numbering method is to start counting from the first amino acid in the amino acid sequence of IL-15 as the first position; preferably, the maternal sequence of IL-15 is shown in SEQ ID NO: 44:

| Combination | **IL15 mutation** |
|---|---|
| 1 | N1D |
| 2 | N4D |
| 3 | D8N |
| 4 | D30N |
| 5 | D61N |
| 6 | E64Q |
| 7 | N65D |
| 8 | Q108E |
| 9 | N1D/D61N |
| 10 | N1D/E64Q |
| 11 | N4D/D61N |
| 12 | N4D/E64Q |
| 13 | D8N/D61N |
| 14 | D8N/E64Q |
| 15 | D61N/E64Q |
| 16 | E64Q/Q108E |
| 17 | N1D/N4D/D8N |
| 18 | D61N/E64Q/N65D |
| 19 | N1D/D61N/E64Q/Q108E |
| 20 | N4D/D61N/E64Q/Q108E |

Preferably, the anti-tumor associated antigen (TAA) and IL-15/IL-15Ra-containing fusion protein comprises an Fc fragment, which selectively eliminates immune effector functions, including but not limited to a combination of the following mutations, according to EU numbering:

| **IgG** | FC Mutate(EU numbing) |
|---|---|
| **IgG1** | L234A,L235A |
| | L234A,L235A,P329G |
| | L234F,L235E,P331S |
| | D265A,N297A |
| | L234F,L235E,N297A |
| | L234F,L235E,D265A |
| | L234A,L235E,P331S |
| | L234A,L235E,N297A |
| | L234A,L235E,D265A |
| | L234AL235AP331S |
| | L234A,L235A,N297A |
| | L234A,L235A,D265A |
| | L235E,D265A,P331S |
| | L235E,N297A,P331S |
| | L235E,N297A |
| | L235AD265AP331S |
| | L235A,N297A,P331S |
| | N297Q |
| | N297A |
| | N297G |
| | A287C, N297G, L306C |
| | R292C, N297G, V302C |
| **hIgG4** | S228P,L235E,P329G |
| | S228P,L235E |
| | S228P,F234A,L235E |
| | S228P,F234A,L235A |
| **IgG2m4** | ASTKGPSVFPLAPCSRSTSESTAALGCLVKDYFPEPVTVSWNSGALT SGVHTFPAVLQSSGLYSLSSVVTVPSSNFGTQTYTCNVDHKPSNTKV DKTVERKCCVECPPCPAPPVAGPSVFLFPPKPKDTLMISRTPEVTCVV VDVSQEDPEVQFNWYVDGVEVHNAKTKPREEQFNSTYRVVSVLTV LHQDWLNGKEYKCKVSNKGLPSSIEKTISKAKGQPREPQVYTLPPSQ EEMTKNQVSLTCLVKGFYPSDIAVEWESNGQPENNYKTTPPVLDSD GSFFLYSRLTVDKSRWQEGNVFSCSVMHEALHNHYTQKSLSLSLGK |
| | N297A |
| | N297Q |
| | N297G |

Preferably, any polypeptide chain of the anti-tumor associated antigen (TAA) and IL-15/IL-15Ra-containing fusion protein has an amino acid sequence shown in SEQ ID NO: 28, or has at least 90% sequence identity with it.

Another purpose of the present invention is to provide a nucleic acid molecule that encodes the anti-tumor associated antigen (TAA) and IL-15/IL-15Ra-containing fusion protein.

Another purpose of the present invention is to provide an application of the anti-tumor associated antigen (TAA) and IL-15/IL-15Ra-containing fusion protein in the preparation of drugs for inhibiting or treating cancer, wherein the cancer is selected from the following cancers or occurs in the following areas: colorectal, breast, ovary, pancreas, stomach, prostate, kidney, cervix, bone marrow cancer, lymphatic cancer, leukemia, thyroid, endometrium, uterus, bladder, neuroendocrine, head and neck, liver, nasopharyngeal, testicle, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, and myelodysplastic syndrome.

Compared to the prior art, the present invention has the following beneficial effects:
(1) The present invention mutates existing anti-CD3 antibody and obtains new anti-CD3 antibody variants.
(2) The present invention also provides antibody molecules with novel structures, which have good biological activity.

### BRIEF DESCRIPTION OF THE DRAWINGS

Figure 1 shows the binding of CD3 antibody SP34 and its mutants to Jurkat cells detected by FACS.
Figure 2 shows the design diagram of the molecular format of bispecific antibody of anti-TAA (tumor associated antigen) and anti-CD3.
Figure 3 shows the design diagram of the molecular format of QP372337241461.
Figure 4 shows the design diagram of the molecular format of QP374437481461.
Figures 5 and 6 show the FACS detection results of CLDN18.2/CD3 bispecific antibody and other molecules binding to CLDN18.2.
Figures 7 and 8 show the FACS detection results of CLDN18.2/CD3 bispecific antibody and other molecules binding to CD3.
Figures 9 and 10 show the results of CLDN18.2/CD3 antibody and other molecules mediated PBMC killing human gastric cancer cells NUGC4 CLDN18.2.
Figure 11 shows the results of CLDN18.2/CD3 antibody and other molecules mediated PBMC killing human lung cancer cells HCC827-CLDN18.2.
Figure 12 shows the inhibitory curve of CLDN18.2/CD3 bispecific antibody molecule inhibiting tumor growth in an *in vivo* animal model of CD3EGD HuGEMM mice subcutaneously transplanted colorectal cancer cell MC38-hCLDN18.2.
Figure 13 shows the weight change curve of mice in each group after administration in an *in vivo* animal model of CD3EGD Hu GEMM mice subcutaneously transplanted colorectal cancer cell MC38-hCLDN18.2.
Figures 14 and 15 show the inhibitory curve of CLDN18.2/CD3 bispecific antibody molecule inhibiting tumor growth in a model of PBMC humanized NOG mice X-CLDN18.2/MIA PaCa-2 subcutaneously transplanted tumor.
Figures 16 to 19 show the structural design diagram of a type of multifunctional anti-TAA/CD3 and IL15/IL15Ra-containing fusion protein.
Figures 20 to 27 show the structural design diagram of another type of multifunctional anti-TAA/CD3 and IL15/IL15Ra-containing protein.
Figure 28 shows the structure design diagram of the TAA/IL15 bifunctional fusion protein activated by tumor targeting T cells/NK cells.
Figure 29 shows the FACS detection results of CLDN18.2/CD3/IL15 multifunctional fusion protein binding to CLDN18.2.
Figure 30 shows the FACS detection results of CLDN18.2/CD3/IL15 and CLDN18.2/IL15 fusion protein binding to CLDN18.2.
Figure 31 shows the FACS detection results of CLDN18.2/CD3/IL15 multifunctional fusion protein binding to CD3.
Figure 32 shows the FACS detection results of CLDN18.2/CD3/IL15 and CLDN18.2/IL15 fusion protein binding to CD3.
Figures 33, 34, and 35 show the results of Mo7e cell proliferation experiment for detecting the activity of IL15 multifunctional fusion protein.
Figures 36 and 37 show the results of CLDN18.2/CD3/IL15 mediated PBMC killing human gastric cancer cells NUGC4 CLDN18.2.
Figure 38 shows the results of CLDN18.2/CD3/IL15 mediated PBMC killing human lung cancer cells HCC827-CLDN18.2.

### BEST MODE OF THE INVENTION

The following is a further explanation of the technical solution of the present invention accompanying with drawings and embodiments.

The experimental methods without specific conditions in this experiment are usually in accordance with conventional conditions or conditions recommended by the manufacturer of raw materials or commodities. Reagents without specific sources are commercially available conventional reagents.

As used herein, "/" refers to "and". For example, anti-tumor associated antigen (TAA)/anti-CD3 bispecific antibody refer to antibody targeting both TAA and CD3 simultaneously.

The term "fusion" refers to connecting components directly by peptide bonds or using linker, or through intermolecular interactions. In a single peptide chain, fusion refers to the direct connection by peptide bonds or the use of linker. A "multifunctional fusion protein" refers to a protein that comprises two or more antigen-binding domains and can bind to two or more different epitopes (such as two, three, or more different epitopes). A multifunctional fusion protein may further comprise cytokines (such as IL-15, IL-15Ra), etc. The "fusion position" refers to the position of functional regions or structural domains in the peptide chain, indicating the connection order of each functional fragment on the peptide chain.

The term "polypeptide" refers to amino acid chain of any length, comprising protein and fragment thereof. The polypeptides are disclosed in amino acid residue sequences in the present invention. Those sequences are written from left to right in the direction from the amino end to the carboxyl end. According to the standard nomenclature, amino acid residue sequences are named by three-letter or single-letter codes, as follows: alanine (Ala, A), arginine (Arg, R), asparagine (Asn, N), aspartate (Asp, D), cysteine (Cys, C), glutathione (Gln, Q), glutamate (Glu, E), glycine (Gly, G), histidine (His, H), isoleucine (I1e, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), and valine (Val, V).

The term "single chain" refers to a molecule comprising amino acids linearly linked by peptide bonds.

The term "variant" or "mutant" refers to a polypeptide or polynucleotide that comprises different amino acids or nucleotides but maintains the basic characteristics. Generally, the differences between variants or between variant and maternal antibody are limited, and the amino acid sequences are very similar in general. In this description, the pre-mutated antibody or antibody fragment is referred to as maternal antibody, and the mutated antibody or antibody fragment is referred to as variant. The variant still has antigen-binding activity.

The term "antibody (Ab)" refers to an immunoglobulin molecule (Ig) that comprises at least one antigen-binding site and can specifically bind to antigen.

The term "antigen" refers to a substance that can induce an immune response in body and specifically bind to antibody. The binding of antibody to antigen relies on the interaction between the two, comprising hydrogen bonds, van der Waals forces, ionic bonds, and hydrophobic bonds. The region on the surface of an antigen to which an antibody binds is an "antigenic determinant" or "epitope". Generally, each antigen has multiple determinants.

The term "antibody" referred in the present invention should be understood in its broadest sense, and encompasses a monoclonal antibody (comprising full-length monoclonal antibody), polyclonal antibody, antibody fragment, and multi-specific antibody comprising at least two different antigen-binding domains (such as bispecific antibody). Antibody also comprises a murine antibody, a humanized antibody, a chimeric antibody, a human antibody, and an antibody from other origins. The antibody of the present invention can originate from any animal, including but not limited to, immunoglobulin molecules of human, non-human primate, mouse, rat, cow, horse, chicken, camel, alpaca, etc. Antibody can comprise additional alterations, such as non-natural amino acids, Fc effector functional mutations, and glycosylation site mutations. Antibody also comprises a post-translational modified antibody, a fusion protein comprising antigenic determinants of antibody, and immunoglobulin molecules comprising any other modification to the antigenic recognition sites, as long as these antibodies exhibit the desired biological activity. In other words, antibody comprises an immunoglobulin molecule and an immune active fragment of immunoglobulin molecule, i.e. a molecule comprising at least one antigen-binding domain.

The basic structure of antibody is a Y-shaped monomer consisting of two identical heavy chains (H) and two identical light chains (L) connected by disulfide bonds. Each chain is composed of 2-5 domains (also known as functional regions) comprising approximately 110 amino acids, with similar sequences but different functions. The amino acid sequences have significant changes near the N-terminus of the light and heavy chains in antibody molecule, forming a structural domain called variable region (V region); the region with relatively constant amino acid sequence near the C-terminus is called the constant region (C region).

The V regions of the heavy and light chains are called VH and VL, respectively. There are three regions in VH and VL with highly variable composition and arrangement of amino acids, known as hypervariable region (HVR). These regions form a spatial conformation complementary to the antigen epitope, which is also known as the complementarity determining region (CDR). The three CDRs of VH are represented by VHCDR1, VHCDR2, and VHCDR3, while the three CDRs of VL are represented by VLCDR1, VLCDR2, and VLCDR3, respectively. A total of 6 CDRs from VH and VL form the antigen binding site. The diversity of amino acids in the CDR region is the molecular basis for the antibody to specifically bind to a large number of different antigens. The composition and arrangement of amino acids other than CDRs in the V region relatively have little change, which are called framework regions (FRs). VH and VL each have four framework regions, which are represented by FR1, FR2, FR3, and FR4, respectively. Each VH and VL is composed of three CDRs and four FRs, arranged from the amino-terminus to the carboxyl-terminus in the following order: FR1, CDR1, FR2, CDR2, FR3, CDR3, FR4.

According to the amino acid sequence of the heavy chain constant region of the antibody, human immunoglobulins can be divided into five classes: IgM, IgG, IgA, IgD, and IgE. It can be further divided into different subtypes (isotypes), for example, human IgG can be divided into IgG1, IgG2, IgG3, and IgG4; IgA can be divided into IgA1 and IgA2. No subtypes have been found for IgM, IgD, and IgE. According to the amino acid sequences of light chains, light chains can be divided as κ chain and λ chain. The antibodies of the present invention may be of any type (such as IgM, IgG, IgA, IgD, IgE) or subtype (such as IgG1, IgG2, IgG3, IgG4, IgA1, IgA2).

The constant regions of heavy and light chains are referred to as CH and CL, respectively. The heavy chain constant region of IgG, IgA, and IgD has three domains of CH1, CH2, and CH3, while the heavy chain constant region of IgM and IgE has four domains of CH1, CH2, CH3, and CH4.

The hinge region is located between CH1 and CH2 and is rich in proline, making it stretchable and bendy, which can change the distance between the Y-shaped arms and facilitate the simultaneous binding of both arms to antigen epitopes.

The term "Fab fragment" is a fragment of antigen binding (Fab), which refers to an antibody fragment comprising VL, VH, CL, and CH1 domains that binds to a single antigen epitope (monovalent). Those skilled in the field know that under certain conditions, certain parts of the antibody molecular chain are easily hydrolyzed into various fragments by proteolytic enzymes. Papain hydrolyzes an antibody molecule into two identical antigen-binding fragments (Fab) and one crystallizable fragment (Fc) from the near N-terminus of the hinge region.

The term "Fd fragment" refers to an antibody fragment consisting of VH and CH1 domains.

The terms "Fc", "Fc segment", "Fc fragment", and "Fc domain" refer to a crystallizable fragment, which has no antigen-binding activity and is the interaction site of antibody with effector molecule or cell surface Fc receptor (FcR). Fc fragments bind to cells with corresponding Fc receptors on their surface, resulting in different biological effects. In ADCC effect (antibody dependent cell mediated cytotoxicity), the Fab segment of the antibody binds to the antigen epitopes of virus infected cells or tumor cells, and its Fc segment binds to the FcR on the surface of killer cells (NK cells, macrophages, etc.) to mediate the direct killing of target cells by killer cells. The Fc fragment comprises constant region polypeptides of the antibody other than the heavy chain constant region CH1, i.e. the constant region domains CH2 and CH3 in the carboxyl end of the heavy chain constant region of human immunoglobulins IgA, IgD, and IgG, and three constant region domains CH2, CH3, and CH4 of the carboxyl end of the heavy chain constant region of human immunoglobulins IgE and IgM. The Fc fragment is usually selected from Human IgG1 Fc, Human IgG2 Fc, Human IgG3 Fc, Human IgG4 Fc or variants thereof, preferably from IgG1 Fc, or Human IgG4 Fc or variants thereof.

Fc may be composed of two chains. As used herein, the two chains of Fc fragment are described as the first Fc chain and the second Fc chain. The first Fc chain and the second Fc chain may be mutated separately, and is not particularly limited in the present invention. The Fc fragments may also refer to a single polypeptide chain in the Fc domain. The Fc segment of the antibody can selectively eliminate immune effector functions, including but not limited to the following combinations of mutations (according to EU numbering).

| **IgG** | FC Mutate(EU numbing) |
|---|---|
| **IgG1** | L234A,L235A |
| | L234A,L235A,P329G |
| | L234F,L235E,P331S |
| | D265A,N297A |
| | L234F,L235E,N297A |
| | L234F,L235E,D265A |
| | L234A,L235E,P331S |
| | L234A,L235E,N297A |
| | L234A,L235E,D265A |
| | L234A,L235A,P331S |
| | L234A,L235A,N297A |
| | L234A,L235A,D265A |
| | L235E,D265A,P331S |
| | L235E,N297A,P331S |
| | L235E,N297A |
| | L235A,D265A,P331S |
| | L235A,N297A,P331S |
| | N297Q |
| | N297A |
| | N297G |
| | A287C, N297G, L306C |
| | R292C, N297G, V302C |
| **hIgG4** | S228P,L235E,P329G |
| | S228P,L235E |
| | S228P,F234A,L235E |
| | S228P,F234A,L235A |
| **IgG2m4** | ASTKGPSVFPLAPCSRSTSESTAALGCLVKDYFPEPVTVSWNSGALT SGVHTFPAVLQSSGLYSLSSVVTVPSSNFGTQTYTCNVDHKPSNTKV DKTVERKCCVECPPCPAPPVAGPSVFLFPPKPKDTLMISRTPEVTCVV VDVSQEDPEVQFNWYVDGVEVHNAKTKPREEQFNSTYRVVSVLTV LHQDWLNGKEYKCKVSNKGLPSSIEKTISKAKGQPREPQVYTLPPSQ EEMTKNQVSLTCLVKGFYPSDIAVEWESNGQPENNYKTTPPVLDSD GSFFLYSRLTVDKSRWQEGNVFSCSVMHEALHNHYTQKSLSLSLGK |
| | N297A |
| | N297Q |
| | N297G |

The mutation designed Fc variants can form spatial filling effects, electrostatic steering, hydrogen bonding, hydrophobic interactions, etc. The interaction between Fc variants helps to form a stable heterodimer. The preferred mutation design is a "Knob in hole" mutation design.

ScFv (single chain antibody fragment), also known as single chain antibody, is formed by connecting the heavy chain variable region and light chain variable region of the antibody through a linker. The "linker" can also connect IL-15 to IL-15Ra, VH of CD3 antibody to VL of that, and ensure correct protein folding and peptide stability. The "linker" is preferably (GGGGS) n, wherein n can be 0, 1, 2, 3, 4, 5, or more. If the linker sequence is too short, it may affect the folding of the higher structure of the two proteins, thereby interfering with each other; if the linker peptide sequence is too long, it also involves immunogenicity issues, since the linker peptide sequence itself is a new antigen.

Tumor-associated antigen (TAA) refers to the antigen molecule present on tumor cells or normal cells, comprising embryonic protein, glycoprotein antigen, squamous cell antigen, etc., commonly used in clinical tumor diagnosis. Tumor-associated antigens are not exclusive to tumor cells, and can be synthesized in trace amounts by normal cells, but they are highly expressed during tumor cell proliferation, hence called "associated antigens". For example, tumor associated antigen can be CD20, CD19, CD30, CD33, CD38, CD40, CD52, slamf7, GD2, CD24, CD47, CD133, CD239, CD276, PD-1, CEA, Epcam, Trop2, TAG72, MUC1, MUC16, mesothelin, folrl, CLDN18.2, PDL1, EGFR, EGFR VIII, C-MET, HER2, FGFR2, FGFR3, PSMA, PSCA, EphA2, ADAM17, 17-A1, NKG2D ligands, MCSP, LGRS, SSEA3, SLC34A2, BCMA, GPNMB, or Glypican-3.

The term "vector" refers to a polynucleotide molecule that can transport another polynucleotide connected to it. One type of vector is a "plasmid", which is a circular double stranded DNA loop that can attach additional DNA segments. Another type of vector is a viral vector, where additional DNA segment can be integrated into the virus genome. Some vectors can replicate autonomously in the host cells that they are introduced in (for example, bacterial vectors with bacterial replication starting points and additive mammalian vectors). Other vectors (such as non-additive mammalian vectors) can be integrated into the host cell genome after being introduced into the host cell, then replicating together with the host genome. In addition, certain vectors can guide the expression of genes that can be operably connected to them. Usually, useful expression vector in recombinant DNA technology is typically in the form of plasmid.

The terms "IL-15" and "IL-15Ra" can refer to their mutants or fragments.

The terms "first" and "second" are only used for descriptive purposes and do not indicate or imply their degree of importance.

The present invention uses the existing CD3 antibody SP34 as the maternal antibody, mutates its variable region, and obtains a CD3 antibody variant. The present invention also provides several protein molecules with different configurations targeting different targets.

The sequence of "IL-15~IL15Ra" can be the following:

SEQ ID NO:42
NWVNVISDLKKIEDLIQSMHIDATLYTESDVHPSCKVTAMKCFLLELQVISCESGDASI
HDTVENLIILANNSLSSNGNVTESGCKECEELEEKNIKEFLQSFVHIVQMFINTSSGGGSGG
GGSGGGGSGGGGSGGGSGGITCPPPMSVEHADIWVKSYSLYSRERYICNSGFKRKAGTC
SLTECVLNKATNVAHWTTPSLKCIR.

The sequence of "IL-15Ra~IL-15" can be the following:

SEQ ID NO:43
ITCPPPMSVEHADIWVKSYSLYSRERYICNSGFKRKAGTCSLTECVLNKATNVAHWTT
PSLKCIRSGGSGGGGSGGGSGGGGSLQNWVNVISDLKKIEDLIQSMHIDATLYTESDVHP
SCKVTAMKCFLLELQVISCESGDASIHDTVENLIILANNSLSSNGNVTESGCKECEELEEKN

IKEFLQSFVHIVQMFINTS.

### I. Obtaining anti-CD3 antibody variants and design of an anti-TAA (tumor associated antigen)/CD3 bispecific antibody format

### Example 1: Obtaining, molecular cloning, transient expression, and protein purification of anti-CD3 antibody variants

1. Molecular cloning: Humanized anti-CD3 antibody SP34 (Wileman et al., 1990; US 8236308) was used for further molecular engineering. Its CDRs were mutated and antibodies with high stability were screened, and three variants were obtained. A single chain antibody VH-VL was constructed by connecting the light and heavy chain variable regions of CD3 parent antibody SP34 and its variants by linkers, and fusing the FC segment of human IgG1 at C-terminus, then installed into the eukaryotic expression vector pQD through molecular cloning. The clone numbers and protein sequence numbers are shown in the table below:

**Table 1: Numbering of CD3 antibody SP34 and its mutant antibodies**

| Protein number | Clone number | Sequence number | Remark |
|---|---|---|---|
| QP3685 | QD3685 | SEQID NO:01 | hSP34 mutant VH-VL-FC |
| QP3689 | QD3689 | SEQID NO:02 | hSP34 mutant VH-VL-FC |
| QP3690 | QD3690 | SEQID NO:03 | hSP34 mutant VH-VL-FC |
| QP3679 (maternal antibody) | QD3679 | SEQID NO:04 | hSP34 VH-VL-FC |

The amino acid sequences are as follows:
SEQ ID NO:01 QD3685 
EVQLLESGGGLVQPGGSLKLSCAASGFTFNTYAMNWVRQAPGKGLEWVARIRSKYN
NYETYYADSVKDRFTISRDDSKNTAYLQMNNLKTEDTAVYYCVRHGNFGNSYVSWFAY
WGQGTLVTVSSGGGGSGGGGSGGGGSELVVTQEPSLTVSPGGTVTLTCRSSTGAVTTSNY
ANWVQQKPGQAPRGLIGGTNLRAPGTPARFSGSLLGGKAALTLSGVQPEDEAEYYCALW
RSGGWVFGGGTKLTVLEPKSSDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQK
SLSLSPGK.
SEQ ID NO:02 QD3689 
EVQLLESGGGLVQPGGSLKLSCAASGFTFNTYAMNWVRQAPGKGLEWVARIRSKYN
NYETYYADSVKDRFTISRDDSKNTAYLQMNNLKTEDTAVYYCVRHSNFGNGYVSWFAY
WGQGTLVTVSSGGGGSGGGGSGGGGSELVVTQEPSLTVSPGGTVTLTCRSSTGAVTTSNY
ANWVQQKPGQAPRGLIGGTNLRAPGTPARFSGSLLGGKAALTLSGVQPEDEAEYYCALW
NKGGWVFGGGTKLTVLEPKSSDKTHTCPPCPAPELLGGPSVFLFPPKPKDTLMISRTPEVT
CVVVDVSHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQK
SLSLSPGK.
SEQ ID NO:03 QD3690 
EVQLLESGGGLVQPGGSLKLSCAASGFTFNTYAMNWVRQAPGKGLEWVARIRSKYN
NYETYYADSVKDRFTISRDDSKNTAYLQMNNLKTEDTAVYYCVRHSNFGNGYVSWFAY
WGQGTLVTVSSGGGGSGGGGSGGGGSELVVTQEPSLTVSPGGTVTLTCRSSTGAVTTSNY
ANWVQQKPGQAPRGLIGGTWLRAPGTPARFSGSLLGGKAALTLSGVQPEDEAEYYCAL
WNSGGWVFGGGTKLTVLEPKSSDKTHTCPPCPAPELLGGPSVFLFPPKPKDTLMISRTPEV
TCVVVDVSHEDPEVKFNWYVDGVEVHNAKTKPREEQYNSTYRVVSVLTVLHQDWLNGK
EYKCKVSNKALPAPIEKTISKAKGQPREPQVYTLPPSRDELTKNQVSLTCLVKGFYPSDIAV
EWESNGQPENNYKTTPPVLDSDGSFFLYSKLTVDKSRWQQGNVFSCSVMHEALHNHYTQ
KSLSLSPGK.
SEQ ID NO:04 QD3679(hSP34 VH-VL-FC)

EVQLLESGGGLVQPGGSLKLSCAASGFTFNTYAMNWVRQAPGKGLEWVARIRSKYN
NYATYYADSVKDRFTISRDDSKNTAYLQMNNLKTEDTAVYYCVRHGNFGNSYVSWFAY
WGQGTLVTVSSGGGGSGGGGSGGGGSELVVTQEPSLTVSPGGTVTLTCRSSTGAVTTSNY
ANWVQQKPGQAPRGLIGGTNKRAPGTPARFSGSLLGGKAALTLSGVQPEDEAEYYCAL
WYSNLWVFGGGTKLTVLEPKSSDKTHTCPPCPAPELLGGPSVFLFPPKPKDTLMISRTPEV
TCVVVDVSHEDPEVKFNWYVDGVEVHNAKTKPREEQYNSTYRVVSVLTVLHQDWLNGK
EYKCKVSNKALPAPIEKTISKAKGQPREPQVYTLPPSRDELTKNQVSLTCLVKGFYPSDIAV
EWESNGQPENNYKTTPPVLDSDGSFFLYSKLTVDKSRWQQGNVFSCSVMHEALHNHYTQ
KSLSLSPGK.

Note: The underlined parts of amino acid sequences represent the heavy chain variable regions of the antibody, the parts with wavy line represent the light chain variable regions, and the italicized parts between the heavy chain variable region and the light chain variable region represent the linker sequence. The arrangement of amino acids in the variable region is FR1-CDR1-FR2-CDR2-FR3-CDR3-FR4, with the bold and bottom highlighted parts representing HCDR1, HCDR3, HCDR3, LCDR1, LCDR2, and LCDR3, respectively. The comparison of maternal antibody and its mutant CDR sequences is shown in the table below:

**Table 2: Comparison of maternal antibody and its mutant CDR sequences**

| Clone number | VHCD R1 | VHCDR2 | VHCDR3 | VLCDR1 | VLCDR2 | VLCDR3 |
|---|---|---|---|---|---|---|
| QD367 9 (matern al antibod y) | TYAM N (SEQ ID NO:29) | RIRSKYNN YATYYAD SVKD (SEQ ID NO:30) | HGNFGNS YVSWFAY (SEQ ID NO:31) | RSSTGAVT TSNYA (SEQ ID NO:32) | GTNKRA P (SEQ ID NO:33) | ALWYSN LWV (SEQ ID NO:34) |
| QD368 5 | TYAM N (SEQ ID NO:29) | RIRSKYNN YETYYAD SVKD (SEQ ID NO:35) | HGNFGNS YVSWFAY (SEQ ID NO:31) | RSSTGAVT TSNYA (SEQ ID NO:32) | GTNLRA P (SEQ ID NO:37) | ALWRSG GWV (SEQ ID NO:39) |
| QD368 9 | TYAM N (SEQ ID NO:29) | RIRSKYNN YETYYAD SVKD (SEQ ID NO:35) | HSNFGNG YVSWFAY (SEQ ID NO:36) | RSSTGAVT TSNYA (SEQ ID NO:32) | GTNLRA P (SEQ ID NO:37) | ALWNKG GWV (SEQ ID NO:40) |
| QD369 0 | TYAM N (SEQ ID NO:29) | RIRSKYNN YETYYAD SVKD (SEQ ID NO:35) | HSNFGNG YVSWFAY (SEQ ID NO:36) | RSSTGAVT TSNYA (SEQ ID NO:32) | GTWLRA P (SEQ ID NO:38) | ALWNSG GWV (SEQ ID NO:41) |

2. Protein expression: The cell density of 293E cells was adjusted to 1 × 10⁶/ml. Plasmid and transfection reagent PEI were prepared, and the amount of plasmids to be transfected were 100µg/100 mL of cells, and the mass ratio of PEI to plasmids was 2:1. The plasmids and PEI were mixed uniformly, then standing for 15 minutes. The mixture of plasmids and PEI was slowly added to 293E cells, and cultured in a shaker at 8% CO₂, 120rpm and 37°C. On the fifth to sixth day of transfection, the cell supernatant was collected after being centrifuged in a horizontal centrifuge at 4700 rpm for 20 minutes for purification.

### 3. Protein purification:

Protein affinity chromatography: the cell culture medium was subjected to high-speed centrifugation and the supernatant was obtained, and affinity chromatography was performed using GE Protein A chromatography column. The equilibrium buffer for chromatography was 1×PBS (pH7.4). After the cell supernatant was loaded and combined, the column was washed with PBS until the ultraviolet light returned to the baseline, then the target protein was eluted with 0.1M glycine (pH3.0), and preserved at a pH adjusted to neutral by Tris. Protein volume exclusion chromatography: the ion-exchange products were concentrated by ultrafiltration for volume exclusion chromatography. For example, GE Superdex200 gel was used for separation to remove possible polymers and other components to obtain target products with high-purity. The purity analysis of the obtained protein can be performed through SDS-PAGE and SEC-HPLC detection. The protein concentration was determined by UV spectrophotometry.

The production and purity of the expressed protein by cells are shown in the table below. QP3679 is the maternal humanized anti-CD3 antibody SP34, with a production of 68.64mg/L, and the mutant anti-CD3 antibody shows higher expression yield than the maternal antibody QP3679, indicating that the mutant has a higher yield and possibly better protein stability. At the same time, the SEC-HPLC detection shows that the protein purity is all good.

**Table 3: Transient Production and Protein Purity of SP34 Antibodies and Their Variants**

| Clone number | Protein number | 293E Transient Expression Production(mg/L) | SEC-HPLC Protein Purity (%) |
|---|---|---|---|
| QD3679 (maternal antibody) | QP3679 | 68.64 | 97.3 |
| QD3685 | QP3685 | 145.86 | 97.2 |
| QD3689 | QP3689 | 142.56 | 97.4 |
| QD3690 | QP3690 | 123.42 | 98.2 |

### Example 2: FACS detection of anti-CD3 antibody variants binding to Jurkat cells naturally expressing CD3

FACS detection of anti-CD3 antibody binding activity: Human lymphocytic leukemia cells (Jurkat cells) express natural CD3. The binding of anti-CD3 antibodies and mutants thereof to Jurkat cells was detected by FACS (fluorescence activated cell sorter). The Jurkat cells were seeded into U-shaped 96-well plate with 1E5/well, washed with cold PBS, and centrifuged at 1200rpm for 3min. After washing, 3% FBS/PBS blocking buffer was added at 200µL/well, and incubated on ice for 1h. After blocking, centrifugation was performed at 1200rpm for 3min, and the supernatant was discarded. Samples with different concentrations were added for incubation on ice for 2h, and washed with cold PBS for three times. PE-anti human FC antibody was added for incubation, which was diluted at a ratio of 1:200, added at 50µl/well and mixed uniformly, then incubated on ice for 1h, and washed with cold PBS for 3 times. Cells were resuspended by 200µl/well PBS, and the average fluorescence values were read on FACS instruments. The results were analyzed using Graphpad prism software.

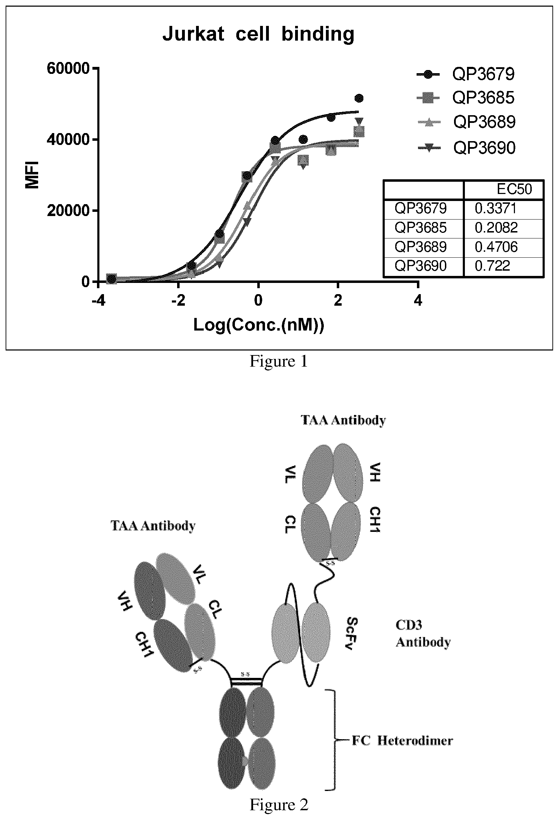

As shown in Figure 1, the activity of the anti-CD3 antibody SP34 and its mutants QP3685, QP3689, and QP3690 binding Jurkat cells were maintained .

### Example 3: Design of an anti-TAA (tumor associated antigen) and anti-CD3 bispecific antibody format

The therapeutic effect of CD3 bispecific antibody is mainly achieved by simultaneously binding to tumor associated antigens (TAA) expressed on tumor cells and CD3 on T cells, cross-linking these two types of cells to form immune synapses, leading to T cell activation and killing tumor cells. Multivalent TAA antibodies can improve selectivity towards targets, especially when the targets are not specific enough. The main direction for improving the safety of CD3 bispecific antibody is to improve the selectivity of TAA, while selecting appropriate CD3 antibody affinity can maintain efficacy and reduce toxic side effects, thereby improving tumor selectivity and reducing targeting of healthy cells.

The molecular format design of the anti-TAA (tumor associated antigen) and anti-CD3 bispecific antibody provided by the present invention is shown in Figure 2.

The anti-TAA and anti-CD3 bispecific antibody molecule shown in Figure 2 comprise: the first monomer (left side of Figure 2) and the second monomer (right side of Figure 2). The first monomer comprises two chains, namely chain 1, which has an Fd fragment comprising VH and CH1; and chain 2, which has a light chain fragment comprising VL and CL, and a first Fc chain. The second monomer also comprises two chains, namely chain 1, which has an Fd fragment comprising VH and CH1; and chain 2, which has a light chain fragment comprising VL and CL, an anti-CD3 antibody fragment (CD3 Antibody in scFv form in Figure 2), and a second Fc chain. In the first or second monomer, the light chain fragment and Fd fragment are paired to form the Fab domain against TAA (used to achieve the function of the TAA Antibody in Figure 2), respectively. Unlike conventional antibody, the light chain fragment of the first monomer is fused with the first Fc chain; meanwhile the N-terminus of the anti-CD3 antibody fragment is fused with the light chain fragment of the second monomer, and the C-terminus of the anti-CD3 antibody fragment is fused with the second Fc chain. The first Fc chain and the second Fc chain are polymerized to form an Fc domain. The Fc domain can be selected from Human IgG1 Fc, Human IgG2 Fc, Human IgG3 Fc, Human IgG4 Fc, or variants thereof.

### Molecular cloning, transient expression, and protein purification of bispecific antibody:

### 1. Molecular cloning:

According to Figure 2, CLDN18.2 was selected as the TAA and an anti-CLDN18.2/CD3 bispecific antibody was designed. The anti-CLDN18.2 antibody sequence can be referred to the sequence of antibody QP14611463 in patent CN202010344676.8. The light chain and heavy chain sequences of SP34 and its variants QP3685, QP3689, and QP3690 were used as anti-CD3 antibody sequence, respectively. The anti-CD3 antibody sequence of QP374537493746 was SP34, and the anti-CD3 antibody sequences of QP090837493746, QP090937474746, and QP091037493746 were selected from QP3685, QP3689, and QP3690. The protein expression and sequence number are shown in the table below.

**Table 4: Clone design of bispecific antibodies**

| Protein number | Clone number | Description | Sequence number |
|---|---|---|---|
| QP374537493746 | QD3745 | QD3745-pQD-anti18.2VL-CL- hSP34 VH-VL-Fc(Knob) | SEQ ID NO:05 |
| | QD3749 | QD3749-pQD-anti18.2VL-CL-Fc(Hole) | SEQ ID NO:06 |
| | QD3746 | QD3746-pQD-antiClaudin18.2VH-CH1 | SEQ ID NO:07 |
| QP090837493746 | QD908 | QD908-pQD-anti18.2VL-CL- hSP34 VH-VL(**QD3685**)-Fc(Knob)(QD3685) | SEQ ID NO:08 |
| | QD3749 | QD3749-pQD-anti18.2VL-CL-Fc(Hole) | SEQ ID NO:06 |
| | QD3746 | QD3746-pQD-antiClaudin18.2VH-CH1 | SEQ ID NO:07 |
| QP090937493746 | QD909 | QD909-pQD-anti18.2VL-CL- hSP34 VH-VL(**QD3689**)-Fc(Knob)(QD3689) | SEQ ID NO:09 |
| | QD3749 | QD3749-pQD-anti18.2VL-CL-Fc(Hole) | SEQ ID NO:06 |
| | QD3746 | QD3746-pQD-antiClaudin18.2VH-CH1 | SEQ ID NO:07 |
| QP091037493746 | QD910 | QD910-pQD-anti18.2VL-CL- hSP34 VH-VL(**QD3690**)-Fc(Knob)(QD3690) | SEQ ID NO:10 |
| | QD3749 | QD3749-pQD-anti18.2VL-CL-Fc(Hole) | SEQ ID NO:06 |
| | QD3746 | QD3746-pQD-antiClaudin18.2VH-CH1 | SEQ ID NO:07 |

At the same time, for comparison, two other forms of anti-CLDN18.2/CD3 bispecific antibody molecules QP372337241461 and QP374437481461 were constructed. According to patent US20200055932A1, CLDN18.2/CD3 bispecific antibody molecule AMG910 from Amgen was constructed, which was numbered QP3693. The protein expression and sequence number are shown in the table below.

**Table 5: Molecular cloning of bispecific antibodies**

| Protein number | Clone number | Description | Sequence number |
|---|---|---|---|
| QP37233724146 1 | QD3723 | QD3723-pQD-anti18.2Fd-hSP34VH-G4Fc(Hole) | SEQ ID NO: 11 |
| | QD3724 | QD3724-pQD-anti18.2Fd-hSP34VL-G4Fc(Knob) | SEQ ID NO: 12 |
| | QD1461 | QD1461-anti18.2LC | SEQ ID NO: 13 |
| QP37443748146 1 | QD3744 | QD3744-pQD-anti18.2Fd-hSP34VH.VL-G4Fc(Kn ob) | SEQ ID NO: 14 |
| | QD3748 | QD3748-pQD-anti18.2Fd(G1)-G4Fc(Hole) | SEQ ID NO:15 |
| | QD1461 | QD1461-anti18.2LC | SEQ ID NO: 13 |
| QP3693 | QD3693 | AMG910 | SEQ ID NO:16 |

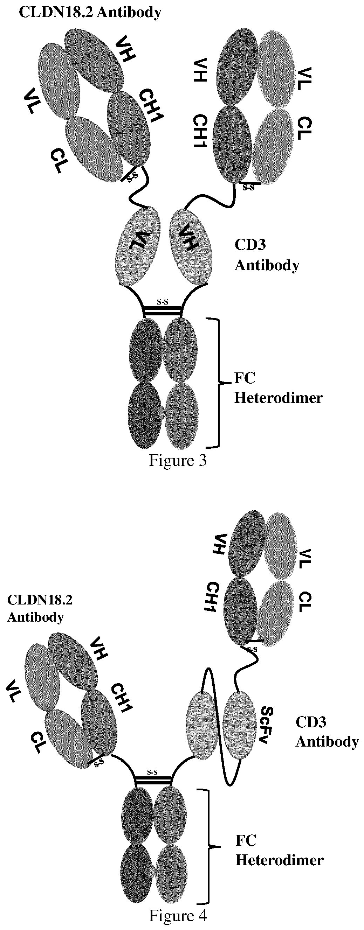

The molecular format design of QP372337241461 is shown in Figure 3. The molecular format design of QP374437481461 is shown in Figure 4.

### 2. Clone construction method

Clones were designed and full-length expression vectors were constructed as shown in Tables 4 and 5. **Primer design:** The online software DNAWorks (v3.2.4) (http://helixweb.nih.gov/dnaworks/) was used to design a number of primers to synthesize fragments comprising the gene fragment needed for recombination. **Fragment splicing:** According to the operational instruction of Primer STAR GXL DNA polymerase from TaKaRa company, multiple primers designed above were used for PCR amplification, and the gene fragments needed for recombination were obtained. Step 1 PCR: 50 µL PCR reaction system comprising 10 µL PrimerSTAR GXL Buffer (5×); 4 µL dNTP Mixture (2.5 mmol · L-1); 1 µL each kind of above-mentioned primers; 1 µL Prime STAR GXL DNA Polymerase. The PCR reaction conditions were 98°C for 2 minutes, 98°C for 20 seconds, 55°C for 15 seconds, 68°C for 30 seconds, and 30 cycles; 68°C for 5 minutes. Step 2 PCR: Using the PCR product in step 1 as a template, the forward and reverse primers were used for PCR amplification under the same conditions as the step 1. The target fragments were amplified by PCR. **Construction and enzyme digestion of expression vector pQD:** the characteristic of some special restriction endonucleases, such as BsmBI, that their recognition sequences differ from and the digestion site was used to design and construct expression vector pQD (with a signal peptide). The vector was digested by BsmBI enzyme, and the glue was cut and recovered for later use. **Recombinant construction of expression vector:** The recombinant target gene fragment and the expression vector pQD (with signal peptide fragments) recovered from BsmBI digestion were added to DH5α competent cells at a ratio of 3:1, and subjected to ice bath at 0°C for 30 minutes, heat shock at 42°C for 90 seconds, then added with 5 times the volume of LB medium, incubated at 37°C for 45 minutes, coated to LB Amp plates, and cultured overnight at 37°C. Single clones were picked for sequencing to obtain various target clones.

### 3. Protein expression

The density of 293E cells was adjusted to 1×10⁶/ml. The plasmids and transfection reagent PEI were prepared, and the amount of plasmids to be transfected was 100ug/100ml cells. The mass ratio of PEI and plasmid is 2:1. The plasmids and PEI were mixed well, then standing for 15 minutes. The mixture of plasmids and PEI was slowly added to 293E cells, and cultured in a shaker at 8% CO₂, 120rpm and 37°C. On the fifth to sixth day of transfection, the cell supernatant was collected after being centrifuged in a horizontal centrifuge at 4700 rpm for 20 minutes for purification.

### 4. Protein purification:

Protein affinity chromatography: The cell culture medium was subjected to high-speed centrifugation and the supernatant was obtained, and affinity chromatography was performed using GE Protein A chromatography column. The equilibrium buffer for chromatography was 1×PBS (pH7.4). After the cell supernatant was loaded and combined, the column was washed with PBS until the ultraviolet light returned to the baseline, then the target protein was eluted with 0.1M glycine (pH3.0), and preserved at a pH adjusted to neutral by Tris. Protein volume exclusion chromatography: the ion-exchange products were concentrated by ultrafiltration for volume exclusion chromatography. For example, GE Superdex200 gel was used for separation to remove possible polymers and other components to obtain target products with high-purity. The purity analysis of the obtained protein can be performed through SDS-PAGE and SEC-HPLC detection. The protein concentration was determined by UV spectrophotometry. The endotoxin was strictly controlled through entire purification process, and the endotoxin content of the purified protein was less than 1EU/mg.

The results show that the CLDN18.2/CD3 bispecific antibodies QP374537493746, QP090837474746, QP090937474746, and QP091037493746 have high cell expression yield, and the purity of HPLC-SEC is higher than 95%, indicating producibility.

### Example 4: FACS detection of bispecific antibody binding activity to CLDN18.2

### Experimental steps:

A cell line CHOS-CLDN18.2 with stable expression of CLDN18.2 were seeded into U-shaped 96-well plate with 1E5/well, washed with cold PBS, and centrifuged at 1200rpm for 3min. After washing, 3% FBS/PBS blocking buffer was added at 200µL/well and incubated on ice for 1h. After blocking, centrifugation was performed at 1200rpm for 3min, and the supernatant was discarded. Samples with different concentrations were added for incubation on ice for 2h, and washed with cold PBS for three times. PE-anti human FC antibody was added for incubation, which was diluted at a ratio of 1:200, added at 50µl/well, and mixed uniformly, then incubated on ice for 1h, and washed with cold PBS for 3 times. Cells were resuspended by 200µl/well PBS, and the average fluorescence values were read on FACS instruments. The results were analyzed using Graphpad prism software.

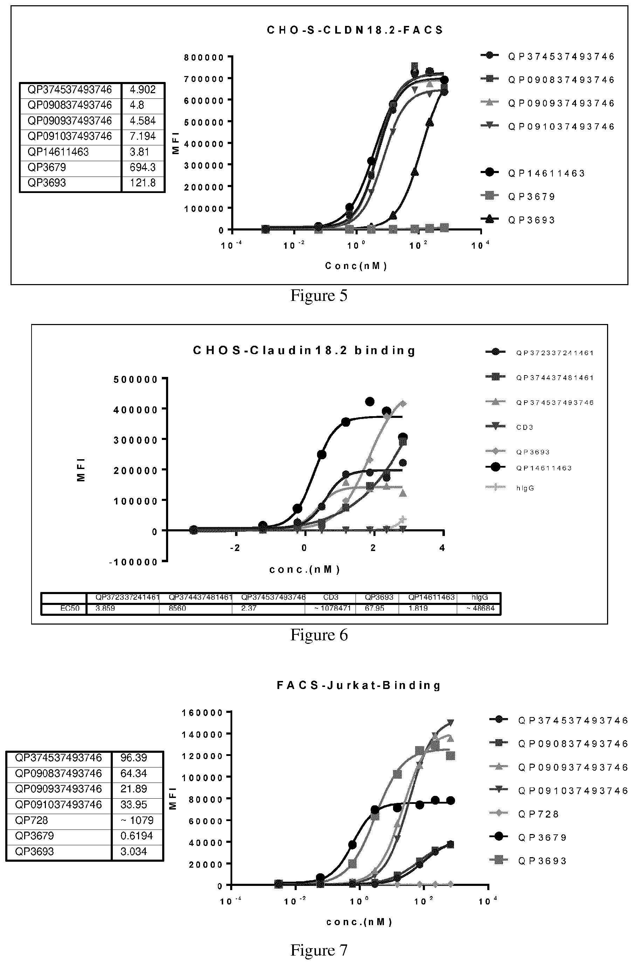

As shown in Figures 5 and 6, the results show that anti-CLDN18.2/CD3 bispecific antibodies QP374537493746, QP090837493746, QP090937493746, and QP091037493746 all bind to CLDN18.2. As shown in Figure 5, the bispecific antibodies QP37453747493746, QP090837474746, QP090937474746, QP0909374746, and QP091037493746 in the form shown in Figure 2 of the present invention have comparable binding affinity to the TAA target CLDN18.2 with the IgG form anti-CLDN18.2 antibody QP14611463, and significantly better than the control antibody AMG910 (1:1 form) QP3693, which meets the design expectation.

### Example 5: FACS detection of bispecific antibody and binding activity to CD3

Human lymphocytic leukemia cells (Jurkat cells) express natural CD3. The binding of anti-CD3 antibodies and mutants thereof to Jurkat cells was detected by FACS. The Jurkat cells were seeded into U-shaped 96-well plate with 1E5/well, washed with cold PBS, and centrifuged at 1200rpm for 3min. After washing, 3% FBS/PBS blocking buffer was added at 200µL/well and incubated on ice for 1h. After blocking, centrifugation was performed at 1200rpm for 3min, and the supernatant was discarded. Samples with different concentrations were added for incubation on ice for 2h, and washed with cold PBS for three times. PE-anti human FC antibody was added for incubation, which was diluted at a ratio of 1:200, added at 50µl/ well, and mixed uniformly, then incubated on ice for 1h, and washed with PBS for 3 times. Cells were resuspended by 200µl/well PBS, and the average fluorescence values were read on FACS instruments. The results were analyzed using Graphpad prism software.

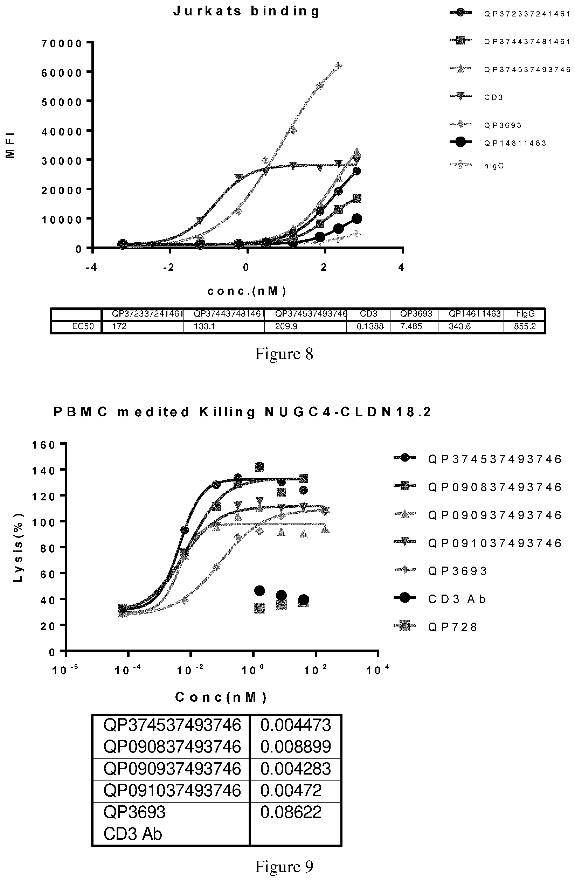

As shown in Figures 7 and 8, the results show that anti-CLDN18.2/CD3 bispecific antibodies QP374537493746, QP090837493746, QP09093747493746, and QP091037493746 all bind to CD3. As shown in Figure 7, the bispecific antibodies QP374537493746, QP090837493746, QP090937474746, and QP091037493746 with the form shown in Figure 2 of the present invention can be divided into two categories in terms of binding affinity to CD3. The binding affinity of QP374537493746 and QP090837493746 to CD3 is weaker, while the binding affinity of QP090937493746 and QP091037493746 is stronger. However, the binding affinity of these two categories of molecules to CD3 is weaker than that of the control antibody AMG910 (1:1 form) QP3693, which meets the expectation that lower affinity of CD3 antibodies will improve the safety of CD3 bispecific antibodies.

### Example 6: anti-CLDN18.2/CD3 bispecific antibody mediated T cell killing of human gastric cancer cells

Experimental method: Human gastric cancer cell line NUGC4-CLDN18.2 stably expressing CLDN18.2 was selected as the target cells. The effector cells were human PBMCs. The E:T ratio was 10:1, and antibodies of different concentrations were added and incubated at 37°C,5% CO₂ for 48 hours. CytoTox 96^{®} Non-Radioactive Cytotoxicity Assay (Promega, G1780-1000 assays) was used to detect LDH in cell culture supernatant and quantify cytotoxicity %. The maximum lysis rate of target cells (100%) is that 1% Triton X-100 treatment of target cells causes cell lysis to release all LDH. Control wells were set for target cell spontaneity, effector cell spontaneity, and target cell + effector cell spontaneity. The data was analyzed according to the formula: % Cytotoxicity=[(Experimental Effector Spontaneous - Target Spontaneous)/(Target Maximum - Target Spontaneous)] ×100.

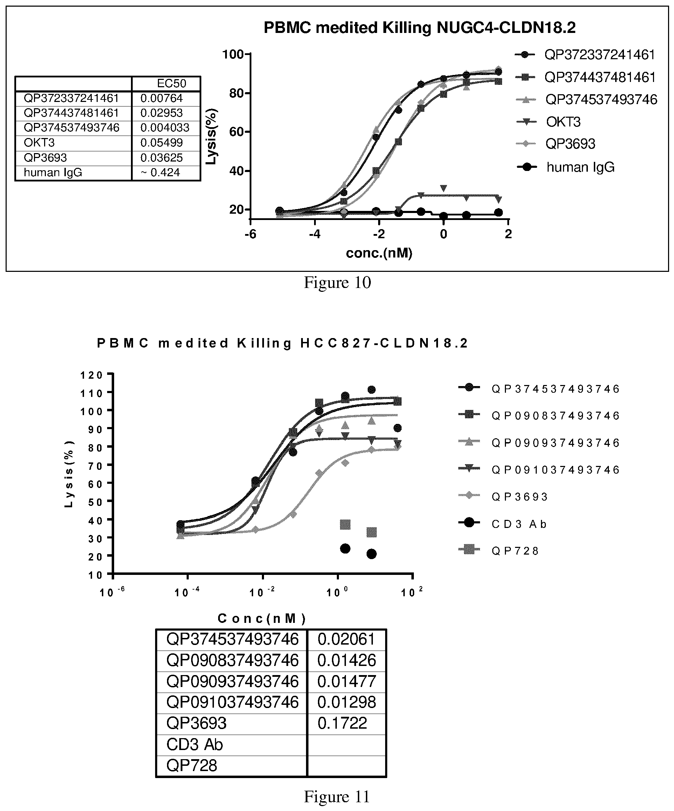

As shown in Figures 9 and 10, QP37453749374746, QP090837474746, QP090937474746, and QP091037493746 all mediate PBMC killing of human gastric cancer cells NUGC4-CLDN18.2. As shown in Figure 9, the activity of PBMC killing human gastric cancer cells NUGC4-CLDN18.2 mediated by the bispecific antibodies QP374537493746, QP090837474746, QP0909374746, and QP091037493746 in the form of Figure 2 of the present invention were stronger than that of the control molecule QP3693 (AMG910). Although the binding affinity to CD3 of the selected CD3 antibody in the present invention is weaker than that of the CD3 antibody of AMG910 molecule (see Figure 7), it does not affect the killing ability against target cells.

### Example 7: anti-CLDN18.2/CD3 bispecific antibody mediated T cell killing of human lung cancer cells

Experimental method: Human lung cancer cell line HCC827-CLDN18.2 stably expressing CLDN18.2 was selected as the target cells. The effector cells were human PBMCs. The E:T ratio was 10:1, and antibodies of different concentrations were added and incubated at 37°C,5% CO₂ for 48 hours. CytoTox 96^{®} Non-Radioactive Cytotoxicity Assay (Promega, G1780-1000 assays) was used to detect LDH in cell culture supernatant and quantify cytotoxicity %. The maximum lysis rate of target cells (100%) is that 1% Triton X-100 treatment of target cells causes cell lysis to release all LDH. Control wells were set for target cell spontaneity, effector cell spontaneity, and target cell + effector cell spontaneity. The data was analyzed according to the formula: % Cytotoxicity = [(Experimental Effector Spontaneous - Target Spontaneous)/(Target Maximum - Target Spontaneous)] ×100.

As shown in Figure 11, CD3 bispecific antibody molecules QP374537493747474746, QP090837474746, QP090937474746, and QP091037493746 all mediate PBMC killing of human lung cancer cells HCC827-CLDN18.2. The activity of PBMC killing human lung cancer cells HCC827-CLDN18.2 mediated by CD3 bispecific antibody molecules QP3745374747474746, QP090837474746, QP090937474746, and QP091037493746 in the form of Figure 2 of the present invention were stronger than that of the control molecule QP3693 (AMG910). The results of NUGC4-CLDN18.2 were repeated on HCC827-CLDN18.2 cells (see Figure 9).

### Example 8: Efficacy of anti-CLDN18.2/CD3 bispecific antibodies in CD3 humanized mouse model

The inhibitory effect of anti-CLDN18.2/CD3 bispecific antibody molecules on tumor growth was evaluated in the CD3 humanized mouse (CD3EDG HuGEMM) model. MC38-hCLDN18.2 colorectal cancer cells in exponential growth phase were collected, and resuspended in PBS to an appropriate concentration for inoculation. Each experimental mouse was subcutaneously inoculated on the right back with 5×10⁶ MC38-hCLDN18.2 cells, and the growth of the tumor was regularly observed. When the tumor grew to an average volume of about 100mm³, the mice were randomly grouped and administered according to the tumor size and mouse weight. The dosage regimen is as follows:

**Table 6: The dosage regimen to evaluate the inhibitory effect of anti-CLDN18.2/CD3 bispecific antibody molecules on tumor growth**

| **Grou p** | **Administration** | **Numbe r of mice** | **Dosage** | | **Administratio n route** | **Dosing frequenc y** |
|---|---|---|---|---|---|---|
| | | | **Drug** | **(mg/kg** ) | | |
| 1 | **Vehicle** | 6 | PBS | - | ip | BIW x 6 |
| 2 | **AMG910** | 6 | AMG910 | 0.7 | ip | BIW x 6 |
| 3 | **QP37453749374 6** | 6 | QP37453749374 6 | 0.6 | ip | BIW x 6 |
| 4 | **QP37233724146 1** | 6 | QP37233724146 1 | 1.2 | ip | BIW x 6 |
| 5 | **QP37443748146 1** | 6 | QP37443748146 1 | 1.2 | ip | BIW x 6 |

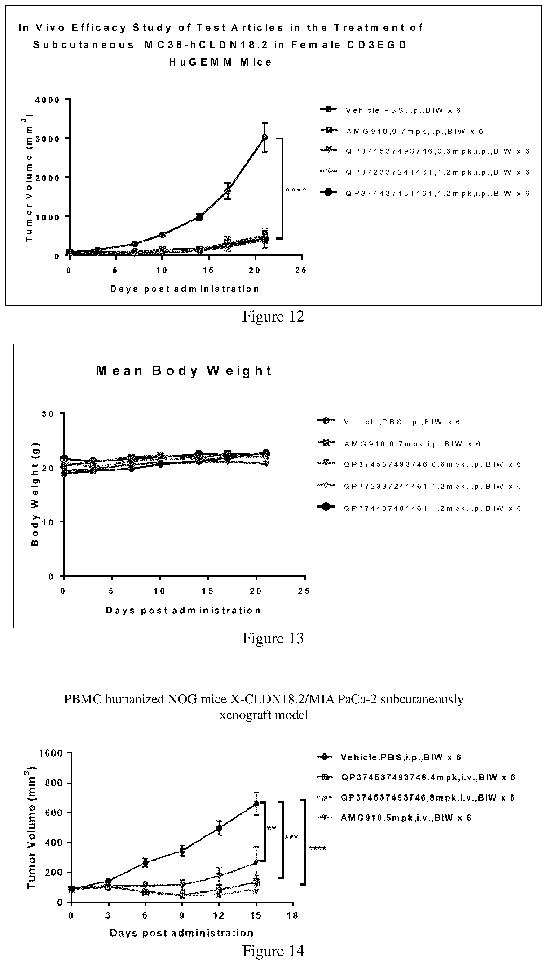

As the dosage regimen shown in the above table, the drugs were administered 6 times and the tumor was measured twice a week. The tumor growth inhibition rate is shown in Table 7, and the tumor inhibition curve is shown in Figure 12. The results show that on the 21st day after administration, the tumor growth inhibition rate (TGI) of QP374537493746 in the low dosage group of 0.6 mpk reaches 89.29%, which was better than or equivalent to the tumor growth inhibition rate (TGI=86.5%) of the control antibody AMG910 in the 0.7 mpk group (the molar concentration of AMG910 in 0.7 mpk group is equivalent to that of QP374537493746 1.2 mpk), indicating that half the dosage of QP374537493746 can achieve the same tumor growth inhibition effect as the control antibody AMG910. Figure 13 shows the body weight changes of mice in each group after administration. The results show that the mice in the administration group remain basically unchanged body weight and grow well, indicating good drug safety.

**Table 7: Inhibition of tumor growth by CLDN18.2/CD3 bispecific antibody molecules in an in vivo animal model of subcutaneous transplantation of colorectal cancer cells MC38-hCLDN18.2 in CD3EGD HuGEMM mice**

| **Gro up** | **Treatment** | **Mean Tumor volume (mm³)** | | **%T GI (D21 )** | **%T/ C (D2 1)** | **P-Value (vs vehicle)** |
|---|---|---|---|---|---|---|
| | | **(Mean±SE M) /grouping** | **(Mean±SEM) / Days post inoculation** | | | |
| 1 | **Vehicle,PBS,i.p.,BIW x 6** | 78.50±5.41(D0) | 3014±911.2(D21) | -- | -- | -- |
| 2 | **AMG910,0.7mpk,i.p.,BIW x 6** | 78.76±6.61(D0) | 475±394.02(D21) | 86.5 0 | 13.5 0 | 0.0001 |
| 3 | **QP374537493746,0.6mpk,i.p.,BIW x 6** | 78.71±6.06(D0) | 393.11±510.1(D21) | 89.29 | 10.71 | 0.0001 |
| 4 | **QP372337241461,1.2mpk,i.p.,BIW x 6** | 78.77±6.47(D0) | 506.37±429.05(D2) | 85.43 | 14.57 | 0.0001 |
| 5 | **QP374437481461,1.2mpk,i.p.,BIW x 6** | 78.67±5.38(D0) | 431.7±274.4(D21) | 87.97 | 12.03 | 0.0001 |

| | | | | | | |
|---|---|---|---|---|---|---|
| *Note: % T/C = (delta T/delta C) × 100, % TGI = 100 - % T/C. | | | | | | |

### Example 9: Efficacy of anti-CLDN18.2/CD3 bispecific antibodies in humanized NOG mouse model

**Experimental objective:** To evaluate the *in vivo* efficacy of the test substance in PBMC humanized NOG mouse X-CLDN18.2/MIA PaCa-2 subcutaneous xenograft model. **Cell culture:** X-CLDN18.2/MIA PaCa-2 cells were cultured *in vitro* under the conditions of medium supplemented with 10% fetal bovine serum, at 37°C, 5% CO₂, and regularly passaged twice a week. When the cells maintained in an exponential growth phase and the cell survival rate was greater than 95%, the cells were collected, counted, and the percentage of live cells was calculated, then the cells were subjected to inoculation. **Animal:** NOG mice, female, 6-8 weeks old, weighing 18-20 grams. A total of 36 animals (24 plus 50%) are required for the experiment. Animals were provided by qualified suppliers. **Tumor inoculation:** 5*10⁶ X-CLDN18.2/MIA PaCa-2 and 2*10⁶ PBMC cells were co-inoculated on the right back of each mouse's neck. At the same time, the experimental animals were labeled with ear labels as the only confirmation mark for subsequent experiments. When the tumor grew and the average volume of the tumor reached approximately 60-100 mm³, the mice were grouped randomly and administrated. The experimental grouping and dosage regimen are shown in Table 8.

**Table 8: The dosage regimen to evaluate the inhibitory effect of anti-CLDN18.2/CD3 bispecific antibody molecules on tumor growth**

| **Grou p** | **Administration** | **Numbe r of mice** | **Dosage** | | **Administratio n route** | **Dosing frequenc y** |
|---|---|---|---|---|---|---|
| | | | **Drug** | **(mg/kg )** | | |
| 1 | **Vehicle** | 6 | PBS | -- | iv | BIW x 5 |
| 2 | **AMG910** | 6 | AMG910 | 5 | iv | BIW x 5 |
| 3 | **QP37453749374 6** | 6 | QP37453749374 6 | 8 | iv | BIW x 5 |
| 4 | **QP37233724146 1** | 6 | QP37233724146 1 | 4 | iv | BIW x 5 |

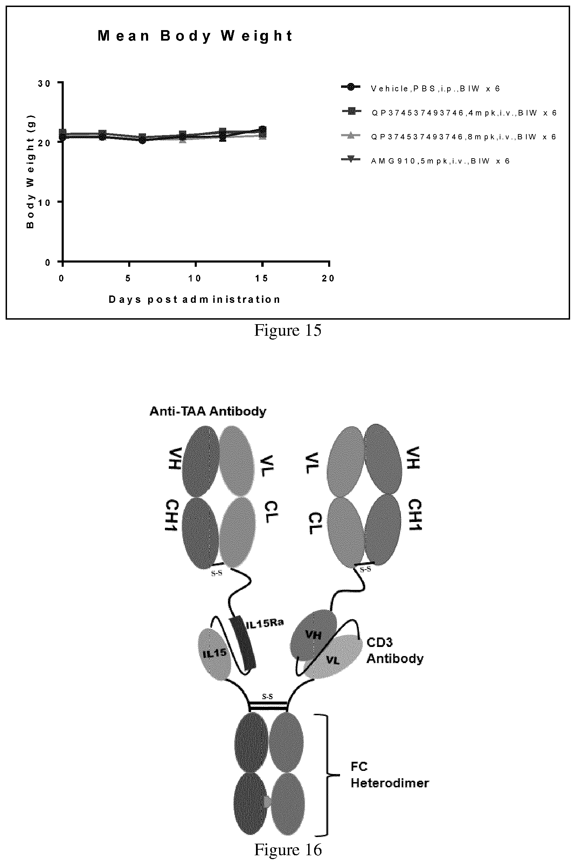

As the dosage regimen shown in the above table, the drugs were administered 5 times and the tumor was measured twice a week. The tumor growth inhibition rate is shown in Table 9, and the tumor inhibition curve is shown in Figure 14. The results show that on the 15^{th} day after administration, the tumor growth inhibition rate (TGI) of QP374537493746 reaches 92.3% in the low dosage group of 4 mpk, and the tumor growth inhibition rate (TGI) in the high dosage group of 8 mpk reaches 99.8%, both are significantly better than that of the control antibody AMG910 in the 5mpk group (TGI=69.3%) (the molar concentration of AMG910 in the 5mpk group is equivalent to that of QP374537493746 in the high-dose group of 8 mpk). Figure 15 shows the body weight changes of mice in each group after administration. The results show that the mice in the administration group has basically no change body weight and grow well, indicating good drug safety.

**Table 9: Inhibition of tumor growth by CLDN18.2/CD3 bispecific antibody molecules inhibits tumor growth in PBMC humanized NOG mouse X-CLDN18.2/MIA PaCa-2 subcutaneous xenograft model**

| Grou p | Treatment | Mean Tumor volume(mm³) | | %T GI (D15 ) | %T/ C (D1 5) | P-Value( vs vehicle) |
|---|---|---|---|---|---|---|
| | | (Mean±SE M) /grouping | (Mean±SEM) / Days post inoculation | | | |
| 1 | Vehicle,PBS,i.v.,BIW x 5 | 89±9(D0) | 659.09±77.03(D 1 5) | - | -- | -- |
| 2 | QP374537493746,4mpk,i.v., BIW x 5 | 89±9(D0) | 133.17±46.00(D1 5) | 92.3 0 | 7.7 | 0.0002 |
| 3 | QP374537493746,8mpk,i.v., BIW x 5 | 89±8(D0) | 89.89±24.56(D15 ) | 99.8 0 | 0.2 | 0.0001 |
| 4 | AMG910,5mpk,i.p.,BIW x 5 | 89±10(D0) | 264.12±107.37(D 15) | 69.3 0 | 30.7 | 0.003 |

| | | | | | | |
|---|---|---|---|---|---|---|
| *Note: % T/C = (delta T/delta C) × 100, % TGI = 100 - % T/C. | | | | | | |

### II. Design of anti-tumor associated antigen (TAA), anti-CD3 antibody, and cytokine IL15/IL15Ra-containing multifunctional fusion protein

Although CD3 bispecific antibodies have shown great potential in hematological tumors, they still face some challenges in the treatment of solid tumors. CD3 bispecific antibody therapy is associated with T cell infiltration and immunosuppression in solid tumor tissue. When there are few infiltrating T cells, it is easy to develop resistance to CD3 bispecific antibody therapy. IL15 is a soluble cytokine that can activate T and NK cells and mediate their proliferation and survival. A multifunctional TAA/CD3/IL15 fusion protein is designed in the present invention, in which the cytokine IL15 component can stimulate the proliferation of immune cells comprising T cells, and alter the immune microenvironment of tumors.

### Example 10: Structure design of anti-TAA (tumor associated antigen), anti-CD3, and IL-15/IL15Ra-containing multifunctional fusion protein

The present invention provides a number of anti-TAA (tumor associated antigen), anti-CD3, and IL15/IL15Ra- containing multifunctional fusion proteins.
1. The first type of multifunctional fusion protein can be seen in Figures 16 to 19, and the fusion protein molecules comprise the first monomer (left part in the figure) and the second monomer (right part in the figure). The first monomer comprises two chains, namely chain 1, which has an Fd fragment (comprising VH and CH1); and chain 2, which has a light chain fragment (comprising VL and CL), a cytokine functional region (comprising IL15 and IL15Ra), and a first Fc chain. The second monomer comprise two chains, namely chain 1, which has an Fd fragment (comprising VH and CH1); and chain 2, which has a light chain fragment (comprising VL and CL), an anti-CD3 antibody fragment (CD3 Antibody in scFv form in the figure), and a second Fc chain. In the first or second monomers, the light chain fragment and Fd fragment are paired to form the Fab domain of anti-TAA (used to achieve the function of anti-TAA antibody in the figure). The N-terminus of the cytokine functional region is fused with the light chain fragment of the first monomer, while the C-terminus is fused with the first Fc chain; the N-terminus of the anti-CD3 antibody fragment is fused with the light chain fragment of the second monomer, and the C-terminus is fused with the second Fc chain. The first Fc chain and the second Fc chain are polymerized to form an Fc domain.

In Figures 16 to 19, the anti-CD3 antibody fragment in scFv form can comprise: a heavy chain variable region (VH), a light chain variable region (VL), and linker connecting the heavy chain variable region and light chain variable region. From the N-terminus to the C-terminus of the peptide chain, the fusion order of amino acids can be "VH (CD3)~VL (CD3)", or "VL (CD3)~VH (CD3)"; wherein "-" represents a linker. The fusion order of amino acid fragments in the cytokine functional region can be "IL-15~IL-15Ra", or "IL-15Ra~IL-15".

The four polypeptide chains of the fusion protein shown in Figure 16 can be described as follows: (1) the first polypeptide chain: VH-CH1; (2) the second polypeptide chain: VL-CL-**IL15Ra-IL15-**the first Fc chain; (3) the third polypeptide chain: VL-CL-**VH (CD3)-VL (CD3)**-the second Fc chain; (4) the fourth polypeptide chain: VH-CH1. "-" represents connecting by a peptide bond or a linker.

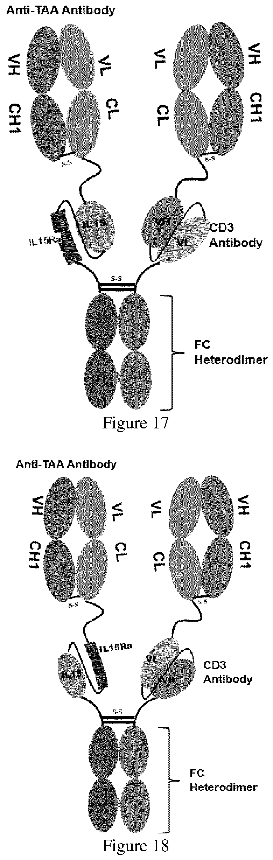

The four polypeptide chains of the fusion protein shown in Figure 17 are described as follows: (1) the first polypeptide chain: VH-CH1; (2) the second polypeptide chain: VL-CL-**IL15-IL15Ra**-the first Fc chain; (3) the third polypeptide chain: VL-CL-**VH (CD3)-VL (CD3)**-second Fc chain; (4) the fourth polypeptide chain: VH-CH1.

The four polypeptide chains of the fusion protein shown in Figure 18 are described as follows: (1) the first polypeptide chain: VH-CH1; (2) the second polypeptide chain: VL-CL-**IL15Ra-IL15-**the first Fc chain; (3) The third polypeptide chain: VL-CL-**VL (CD3) VH (CD3)**-second Fc chain; (4) The fourth polypeptide chain: VH-CH1.

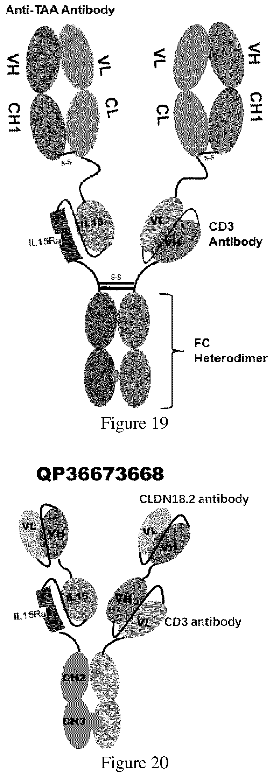

The four polypeptide chains of the fusion protein shown in Figure 19 are described as follows: (1) the first polypeptide chain: VH-CH1; (2) The second polypeptide chain: VL-CL-**IL15-IL15Ra-**the first Fc chain; (3) The third polypeptide chain: VL-CL-**VL (CD3) VH (CD3)**-the second Fc chain; (4) The fourth polypeptide chain: VH-CH1.

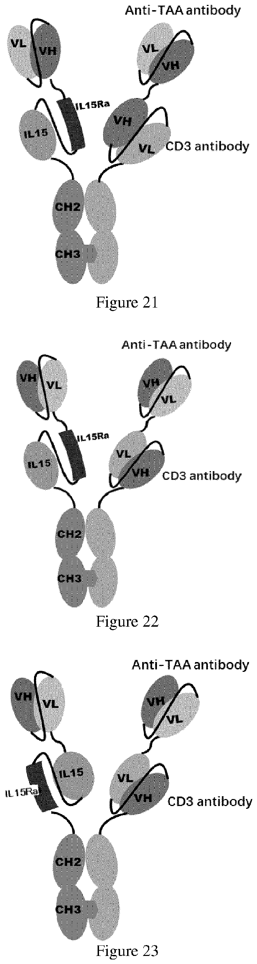

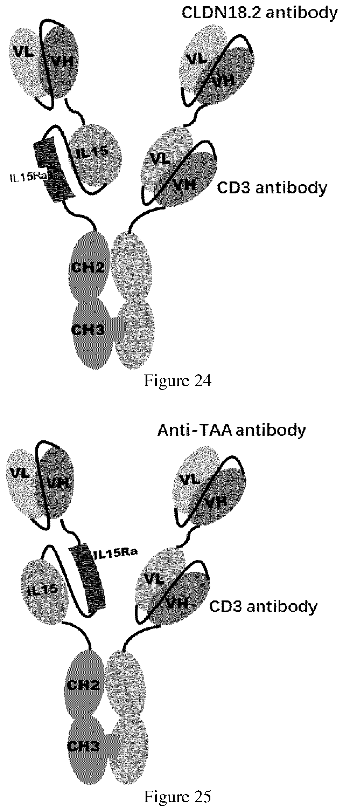

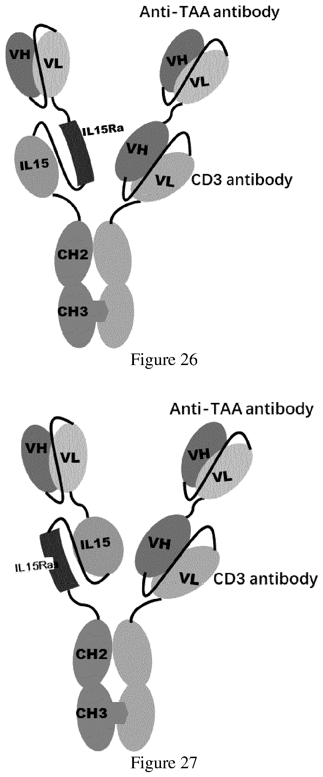

2. The second type of multifunctional fusion protein can be seen in Figures 20 to 27, and the protein molecules comprise the first monomer (left part in the figure) and the second monomer (right part in the figure). The first monomer comprises: an anti-TAA antibody fragment (in scFv form, comprising VH and VL domains), a cytokine functional region (comprising IL15 and IL15Ra), and a first Fc chain; the second monomer comprises: an anti-TAA antibody fragment (in scFv form, comprising VH and VL domains), an anti-CD3 antibody fragment (CD3 Antibody in the figure in scFv form), and a second Fc chain. The N-terminus of the cytokine functional region is fused with the anti-TAA antibody fragment of the first monomer, while the C-terminus is fused with the first Fc chain; the N-terminus of the anti-CD3 antibody fragment is fused with the anti-TAA antibody fragment of the second monomer, and the C-terminus is fused with the second Fc chain. The first Fc chain and the second Fc chain are polymerized to form an Fc domain.

Similar to the first type of multifunctional fusion protein, the anti-CD3 antibody fragment in scFv form can comprise: a heavy chain variable region (VH), a light chain variable region (VL), and a linker connecting heavy chain variable region and light chain variable region. The fusion order of amino acids in the anti-CD3 antibody fragment from the N-terminus to the C-terminus of the peptide chain can be "VH (CD3)~VL (CD3)", or "VL (CD3)~VH (CD3)"; wherein "-" represents a linker. The fusion order of amino acid fragments in the cytokine functional region can be "IL-15~IL-15Ra", or "IL-15Ra~IL-15". In addition, the anti-TAA antibody fragment in scFv form comprises: a heavy chain variable region (VH), a light chain variable region (VL), and a linker connecting the heavy chain variable region and the light chain variable region; the amino acid fusion sequence of the anti-TAA antibody fragment from the N-terminus to the C-terminus of the peptide chain can be "VH (TAA)~VL (TAA)", or "VL (TAA)-VH (TAA)"; wherein "∼" represents a linker.

The domains of the antibody molecules shown in Figures 20 to 27 share the same amino acid sequence, and differ in fusion order or configuration. Taking Figure 20 as an example, the two polypeptide chains of the fusion protein are described as follows: (1) the first polypeptide chain: VL (TAA) - VH (TAA) - IL15- IL15Ra - the first Fc chain; (2) The second polypeptide chain: VL (TAA) - VH (TAA) - VH (CD3) - VL (CD3) - the second Fc chain.

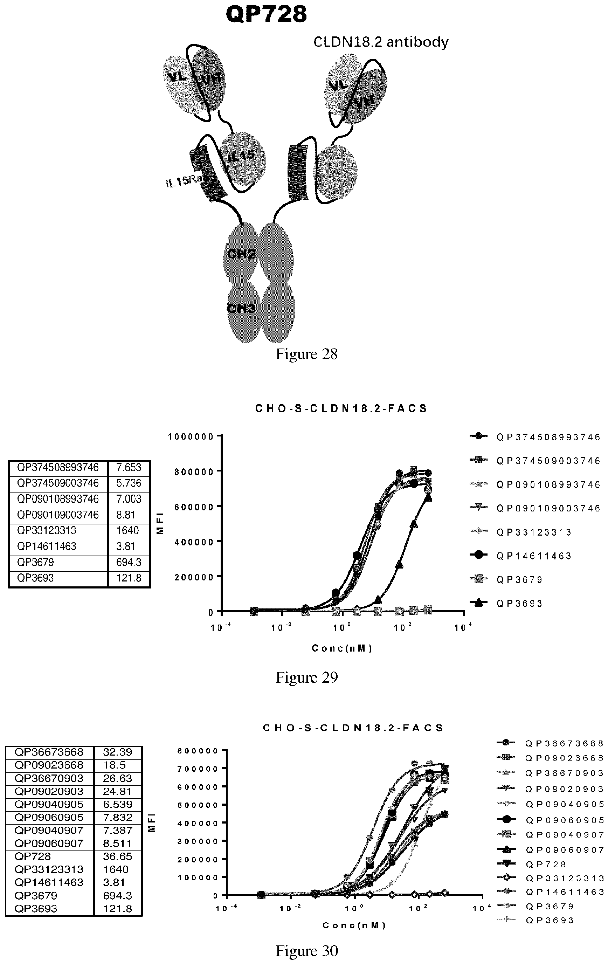

3. The present invention also provides a TAA/IL15 bifunctional fusion protein activated by tumor-targeted T/NK cell, whose structural form can be seen in Figure 28. Compared to the second type of multifunctional fusion protein, the difference is that the anti-CD3 antibody fragment is replaced with a cytokine functional region (comprising IL15 and IL15Ra). In Figure 28, the protein molecule has a symmetrical structural form.

### Example 11: Molecular cloning, transient expression, and protein purification of CLDN18.2/CD3/IL15 multifunctional fusion protein

### 1. Molecular cloning:

According to Example 10, a multifunctional fusion protein CLDN18.2/CD3/IL15 or CLDN18.2/IL15 was designed. For the CLDNI8.2 antibody, please refer to the sequence of the antibody QP14611463 in the patent document CN202010344676.8. The anti-CD3 antibody has uses SP34 light and heavy chain sequences. The protein expression and sequence numbers are shown in the table below.

**Table 10: Clone design of multifunctional fusion proteins**

| Protein number | Clone number | Description | Sequence number |
|---|---|---|---|
| QP37450899374 6 | QD3745 | QD3745-pQD-anti18.2VL-CL- hSP34 VH-VL-Fc(Knob) | SEQ ID NO:05 |
| | QD0899 | QD899-pQD-anti18.2VL-CL-IL15Ra-IL15-Fc(Hole ) | SEQ ID NO:17 |
| | QD3746 | QD37 46- pQD-antiClaudin 18 .2VH -CH 1 | SEQ ID NO:07 |
| QP37450900374 6 | QD3745 | QD3745-pQD-anti18.2VL-CL- hSP34 VH-VL-Fc(Knob) | SEQ ID NO:01 |
| | QD0900 | QD900-pQD-anti18.2VL-CL-IL15-IL15Ra-Fc(Hole ) | SEQ ID NO:18 |
| | QD3746 | QD37 46- pQD-antiClaudin 18 .2VH -CH 1 | SEQ ID NO:07 |
| QP09010899374 6 | QD0901 | QD901-pQD-anti18.2VL-CL- hSP34 VL-VH-Fc(Knob) | SEQ ID NO:19 |
| | QD0899 | QD899-pQD-anti18.2VL-CL-IL15Ra-IL15-Fc(Hole ) | SEQ ID NO:17 |
| | QD3746 | QD37 46- pQD-antiClaudin 18 .2VH -CH 1 | SEQ ID NO:07 |
| QP09010900374 6 | QD0901 | QD901-pQD-anti18.2VL-CL- hSP34 VL-VH-Fc(Knob) | SEQ ID NO: 19 |
| | QD0900 | QD900-pQD-anti18.2VL-CL-IL15-IL15Ra-Fc(Hole ) | SEQ ID NO:18 |
| | QD3746 | QD37 46- pQD-antiClaudin 18 .2VH -CH 1 | SEQ ID NO:07 |
| QP36673668 | QD3667 | QD3667-antiCLDN18.2 VL-VH-IL15-IL15Ra-IgG4 FC(Hole)-flag: | SEQ ID NO:20 |
| | QD3668 | QD3668- antiCLDN18.2 VL-VH-Hsp34 VH-VL-IgG4 FC(Knob)-his: | SEQ ID NO:21 |
| QP09023668 | QD0902 | QD902-antiCLDN18.2 VL-VH-IL15Ra-IL15-IgG4 FC(Hole)-flag: | SEQ ID NO:22 |
| | QD3668 | QD3668- antiCLDN18.2 VL-VH-Hsp34 VH-VL-IgG4 FC(Knob)-his: | SEQ ID NO:21 |
| QP36670903 | QD3667 | QD3667-antiCLDN18.2 VL-VH-IL15-IL15Ra-IgG4 FC(Hole)-flag: | SEQ ID NO:20 |
| | QD0903 | QD903- antiCLDN18.2 VL-VH-Hsp34 VL-VH-IgG4 FC(Knob)-his: | SEQ ID NO:23 |
| QP09020903 | QD0902 | QD902-antiCLDN18.2 VL-VH-IL15Ra-IL15-IgG4 FC(Hole)-flag: | SEQ ID NO:22 |
| | QD0903 | QD903- antiCLDN18.2 VL-VH-Hsp34 VL-VH-IgG4 FC(Knob)-his: | SEQ ID NO:23 |
| QP09040905 | QD0904 | QD904-antiCLDN18.2 VH-VL-IL15-IL 15Ra-IgG4 FC(Hole)-flag: | SEQ ID NO:24 |
| | QD0905 | QD905- antiCLDN18.2 VH-VL-Hsp34 VH-VL-IgG4 FC(Knob)-his: | SEQ ID NO:25 |
| QP09060905 | QD0906 | QD906-antiCLDN18.2 VH-VL-IL15Ra-IL15-IgG4 FC(Hole)-flag: | SEQ ID NO:26 |
| | QD0905 | QD905- antiCLDN18.2 VH-VL-Hsp34 VH-VL-IgG4 FC(Knob)-his: | SEQ ID NO:25 |
| QP09040907 | QD0904 | QD904-antiCLDN18.2 VH-VL-IL15-IL15Ra-IgG4 FC(Hole)-flag: | SEQ ID NO:24 |
| | QD0907 | QD907- antiCLDN18.2 VH-VL-Hsp34 VL-VH-IgG4 FC(Knob)-his: | SEQ ID NO:27 |
| QP09060907 | QD0906 | QD906-antiCLDN18.2 VH-VL-IL15Ra-IL15-IgG4 FC(Hole)-flag: | SEQ ID NO:26 |
| | QD0907 | QD907- antiCLDN18.2 VH-VL-Hsp34 VL-VH-IgG4 FC(Knob)-his: | SEQ ID NO:27 |
| QP728 | QD0728 | QD728-Pqd-CLDN18.2 VL-VH-IL 15-IL 15Ra-FC(IgG4) | SEQ ID NO:28 |

At the same time, the IL15/IL15Ra-Fc of Jiangsu Hengrui Pharmaceuticals Co.,Ltd. was constructed as a control, and the IL15/IL15Ra-Fc protein was numbered QP33123313.

Clones were designed according to Table 10 and full-length expression vectors were constructed. The methods for clone construction, protein expression, and protein purification can be found in Example 3.

### Example 12: FACS detection of the binding activity of CLDN18.2/CD3/IL15 multifunctional fusion protein to CLDN18.2

Experimental steps: A cell line CHOS-CLDN18.2 with stable expression of CLDN18.2 were seeded into U-shaped 96-well plate with 1E5/well, washed with cold PBS, and centrifuged at 1200rpm for 3min. After washing, 3% FBS/PBS blocking buffer was added at 200µL/well and incubated on ice for 1h. After blocking, centrifugation was performed at 1200rpm for 3min, and the supernatant was discarded. Samples with different concentrations were added for incubation on ice for 2h, and washed with cold PBS for three times. PE-anti human FC antibody was added for incubation, which was diluted at a ratio of 1:200, added at 50µl/well, and mixed uniformly, then incubated on ice for 1h, and washed with cold PBS for 3 times. Cells were resuspended by 200µl/well PBS, and the average fluorescence values were read on FACS instruments. The results were analyzed using Graphpad prism software. As shown in Figure 29 and Figure 30, the results show that both CLDN18.2/CD3/IL15 and CLDN18.2/IL15 multifunctional fusion proteins bind to the TAA target CLDN18.2, and their affinity is better than that of the AMG910 analog (1:1 form) QP3693, which is in line with expectations.

### Example 13: FACS detection of the binding activity CLDN18.2/CD3/IL15 multifunctional fusion protein to CD3

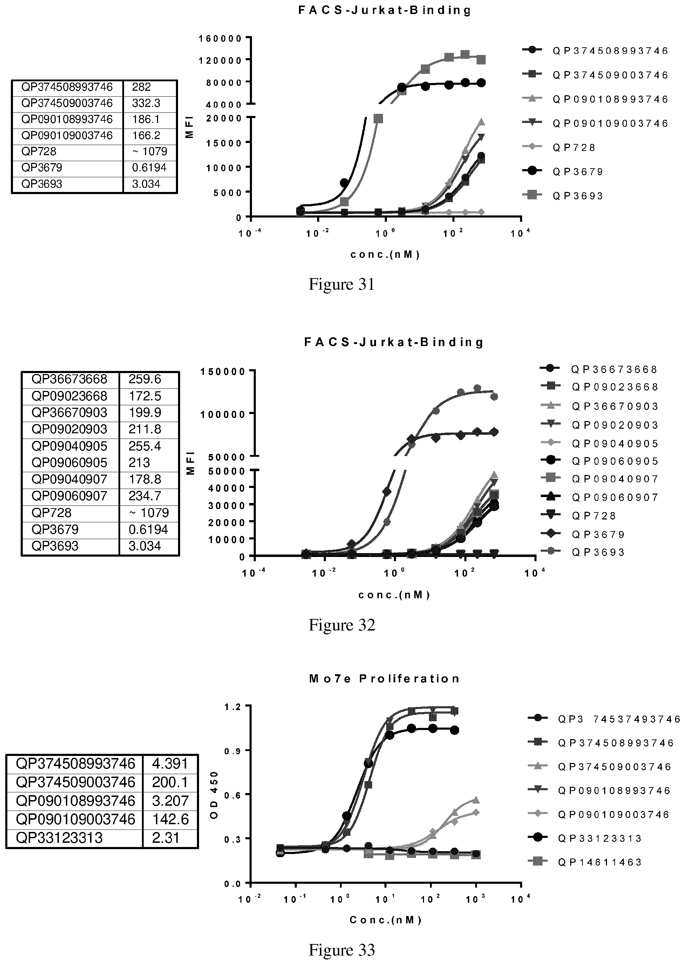

Human lymphocytic leukemia cells (Jurkat cells) express natural CD3. The binding of anti-CD3 antibodies and mutants thereof to Jurkat cells was detected by FACS. The Jurkat cells were seeded into U-shaped 96-well plate with 1E5/well, washed with cold PBS, and centrifuged at 1200rpm for 3min. After washing, 3% FBS/PBS blocking buffer was added at 200µL/well and incubated on ice for 1h. After blocking, centrifugation was performed at 1200rpm for 3min, and the supernatant was discarded. Samples with different concentrations were added for incubation on ice for 2h, and washed with cold PBS for three times. PE-anti human FC antibody was added for incubation, which was diluted at a ratio of 1:200, added at 50µl/well, and mixed uniformly, then incubated on ice for 1h, and washed with cold PBS for 3 times. Cells were resuspended by 200µl/well PBS, and the average fluorescence values were read on FACS instruments. The results were analyzed using Graphpad prism software. As shown in Figures 31 and 32, the results show that all CLDN18.2/CD3/IL15 multifunctional fusion protein bind to CD3 with a weaker affinity than the control molecule AMG910 (QP3693). This is consistent with the inventor's expectation that the low affinity of CD3 antibodies will improve the safety of CD3 bispecific antibodies.

### Example 14: Evaluation of the activity of multifunctional fusion protein IL15 by Mo7e cell proliferation assay

Mo7e (human giant cell leukemia cell line) is a cytokine growth dependent cell expressing IL-15Rβγ. Previous studies have shown that resting NK and naive T cells express moderate affinity IL-15Rβγ phenotype, and the results of cytokine IL15/IL15Ra on the proliferation of Mo7e (IL-15Rβγ) cells are consistent with those of unstimulated PBMC cells (Mol Cancer Ther; 11 (6) June 2012). Mo7e cell proliferation assay was used in the present invention to evaluate the activity of multifunctional fusion protein IL15. The method and results are as follows:
**Experimental reagent:** Mo7e cells (human giant cell leukemia cell line) were purchased from the Cell Resource Center of the Institute of Basic Medicine, Chinese Academy of Medical Sciences; Cell Proliferation and Toxicity Test Kit (CCK-8) were purchased from Mei lun Biotech, catalog number MA0218; recombinant human GM-CSF was purchased from Properotech, catalog number 300-03; human IgG was purchased from Sigma, catalog number I4506; and other antibodies come from internal preparation.
**Experimental method:** Mo7e cells were cultured with RPMI1640 medium comprising 10% FBS, 2mM L-glutamine, and 8ng/ml GM-CSF in a 5% CO₂ incubator at 37°C; Mo7e cells were collected, and centrifuged at 800rpm for 5 minutes to discard the supernatant. The cells were washed twice with RPMI1640 culture medium without GM-CSF, and resuspended in RPMI1640 culture medium without GM-CSF and counted, then seeded in a 96 well plate at 2×10⁴ cells, 80 µl per well, and incubated in a 5% CO₂ incubator at 37°C for 1 hour. Each medicinal culture medium to be tested was diluted with 4 times for gradients, added at 20µl per cell and mixed well with cell suspension, then incubated in a 5% CO₂ incubator at 37°C for 3 days; CCK-8 reagent was added to the 96 well plate 10µl per well, and incubated in a 5% CO₂ incubator at 37°C for 4 hours; The 96 well plate was taken out and the absorbance value was measured at a wavelength of 450nm in a microplate reader.

The experimental results are shown in Figure 33, Figure 34 and Figure 35. The results show that CLDN18.2/CD3/IL15 and CLDN18.2/IL15 multifunctional fusion proteins with different forms all have IL15 activity, and the activity varies from strong to weak according to different molecular designs. Different formats of construction produce molecules with different IL15 activity, which provides an opportunity to select the best therapeutic window.

### Example 15: CLDN18.2/CD3/IL15 multifunctional fusion protein mediated T cell killing of human gastric cancer cells

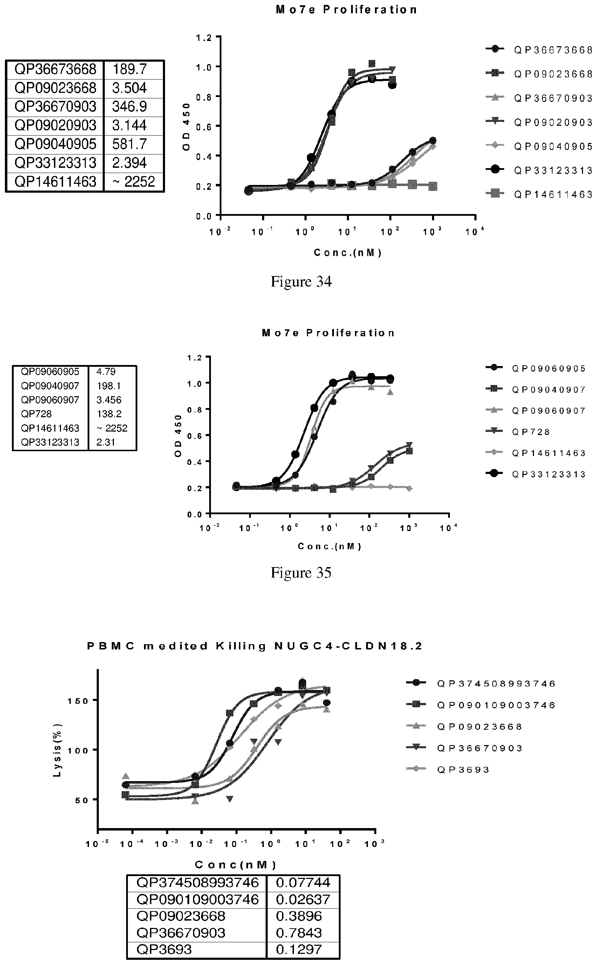

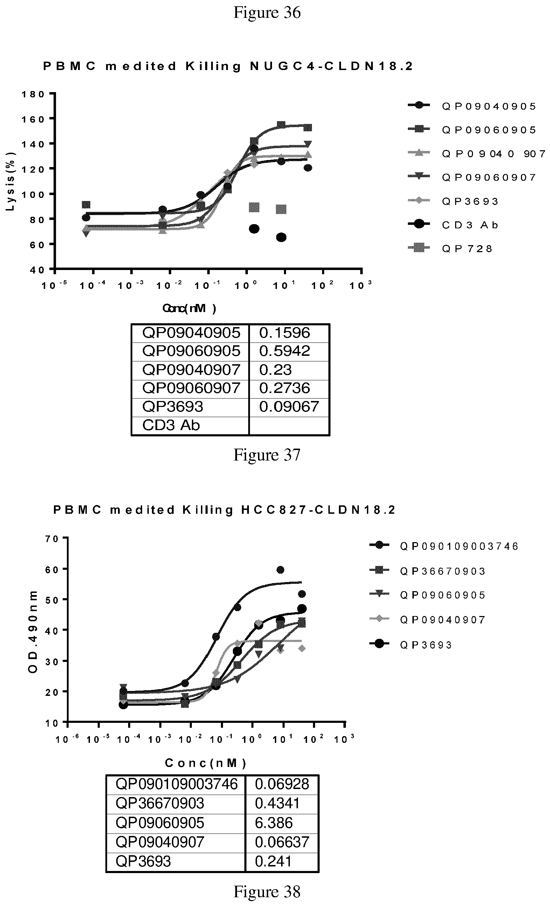

**Experimental method:** Human gastric cancer cell line NUGC4-CLDN18.2 stably expressing CLDN18.2 was selected as the target cells. The effector cells were human PBMCs. The E:T ratio was 10:1, and antibodies of different concentrations were added and incubated at 37°C, 5% CO₂ for 48 hours. CytoTox 96^{®} Non-Radioactive Cytotoxicity Assay (Promega, G1780-1000 assays) was used to detect LDH in cell culture supernatant and quantify cytotoxicity %. The maximum lysis rate of target cells (100%) is that 1% Triton X-100 treatment of target cells causes cell lysis to release all LDH. Control wells were set for target cell spontaneity, effector cell spontaneity, and target cell + effector cell spontaneity. The data was analyzed according to the formula: % Cytotoxicity=[(Experimental Effector Spontaneous - Target Spontaneous)/(Target Maximum - Target Spontaneous)] ×100. The results are shown in Figure 36 and Figure 37. QP374508993746, QP090109003746, QP09023688, QP36670903, QP09040905, QP09060905, QP09040907 and QP09060907 all can mediate PBMC to kill human gastric cancer cell NUGC4-CLDN 18.2 cells. Among them, the activity of PBMC killing the human gastric cancer cell NUGC4-CLDN18.2 mediated by QP374508993746 and QP090109003746 was significantly better than that of control molecule AMG910. The activity of PBMC killing the human gastric cancer cell NUGC4-CLDN18.2 mediated by other molecules was comparable to that of control molecule AMG910.

### Example 16: CLDN18.2/CD3/IL15 multifunctional fusion protein mediated T cell killing of human lung cancer cells

**Experimental method:** Human lung cancer cell line HCC827-CLDN18.2 stably expressing CLDN18.2 was selected as the target cells. The effector cells were human PBMCs. The E:T ratio was 10:1, and antibodies of different concentrations were added and incubated at 37°C, 5% CO₂ for 48 hours. CytoTox 96^{®} Non-Radioactive Cytotoxicity Assay (Promega, G1780-1000 assays) was used to detect LDH in cell culture supernatant and quantify cytotoxicity %. The maximum lysis rate of target cells (100%) is that 1% Triton X-100 treatment of target cells causes cell lysis to release all LDH. Control wells were set for target cell spontaneity, effector cell spontaneity, and target cell + effector cell spontaneity. The data was analyzed according to the formula: % Cytotoxicity=[(Experimental Effector Spontaneous - Target Spontaneous)/(Target Maximum - Target Spontaneous)] ×100. As shown in Figure 38, the results show that QP090109003746, QP36670903, QP09040905, QP09060905 and QP09040907 all have the activity of mediating PBMC to kill human lung cancer cell HCC827-CLDN18.2. Among them, the activity of PBMC killing human lung cancer cell HCC827-CLDN18.2 mediated by QP090109003746 was significantly better than that of control molecule AMG910. The activity of PBMC killing human lung cancer cell HCC827-CLDN18.2 mediated by other molecules was comparable to that of control molecule AMG910.

In addition, in some other embodiments, the anti-CD3 antibody fragment was selected from the antibody light and heavy chain sequences of SP34 variants QP3685, QP3689, and QP3690 respectively, and the proteins were designed, cloned, expressed, and purified as shown in Table 10 of Example 11. The results show that the molecules constructed using the light and heavy chain sequences of QP3685, QP3689 and QP3690 also have CLDN18.2 binding activity and IL15 activity, while the anti-CD3 molecules also have CD3 binding activity, and have the effect of mediating T cells to kill human gastric cancer cells or lung cancer cells.

Although the contents of the present invention have been described in detail with reference to the above preferred embodiments, it should be recognized that the above description should not be considered a limitation of the present invention. Various modifications and alternatives to the present invention will be obvious to those skilled in the art upon reading the foregoing . Therefore, the scope of protection of the present invention shall be limited by the appended claims.

## Claims

1. An anti-CD3 antibody variant, comprising a heavy chain variable region and a light chain variable region; the heavy chain variable region comprises: VHCDR1, VHCDR2, and VHCDR3; the light chain variable region comprises: VLCDR1, VLCDR2, and VLCDR3; the amino acid sequence of the anti-CD3 antibody comprises VHCDR1, VHCDR2, VHCDR3, VLCDR1, VLCDR2, and VLCDR3 shown in SEQ ID NO: 29-34; wherein:
the mutation site of the anti-CD3 antibody variant comprises any one or more of the following sites:
position 10 in SEQ ID NO: 30,
positions 2 and 7 in SEQ ID NO: 31,
positions 3 and 4 in SEQ ID NO: 33,
positions 4, 5, 6, and 7 in SEQ ID NO: 34.

2. The anti-CD3 antibody variant of claim 1, wherein the mutation refers to amino acid substitution.

3. The anti-CD3 antibody variant of claim 1, wherein the mutation site of the variant comprises any one or more of the following sites:
A in position 10 substituted with E in the sequence of SEQ ID NO: 30,
G in position 2 substituted with S, and S in position 7 substituted with G in the sequence of SEQ ID NO: 31,
N in position 3 substituted with W, and K in position 4 substituted with L in the sequence of SEQ ID NO: 33,
Y in position 4 substituted with N or R, S in position 5 substituted with K, N in position 6 substituted with G, and L in position 7 substituted with G in the sequence of SEQ ID NO: 34.

4. The anti-CD3 antibody variant of claim 1, wherein
the amino acid sequence of VHCDR2 is shown in SEQ ID NO: 35; or
the amino acid sequence of VHCDR3 is shown in SEQ ID NO: 36; or
the amino acid sequence of VLCDR2 is shown in SEQ ID NO: 37 or 38; or
the amino acid sequence of VLCDR3 is shown in SEQ ID NO: 39, 40 or 41.

5. The anti-CD3 antibody variant of claim 1, wherein the amino acid sequences of VHCDR1, VHCDR2, VHCDR3, VLCDR1, VLCDR2, and VLCDR3 are shown in any one of the following groups of sequences:
(1) SEQ ID NOs: 29, 35, 31, 32, 37, 39;
(2) SEQ ID NOs: 29, 35, 36, 32, 37, 40;
(3) SEQ ID NOs: 29, 35, 36, 32, 38, 41.

6. The anti-CD3 antibody variant of claim 5, wherein the heavy chain variable region and/or light chain variable region of the antibody variant are selected from the heavy chain variable region and/or light chain variable region has the sequence shown in or have at least 90% sequence identity with SEQ ID NO: 1, 2, or 3.

7. The anti-CD3 antibody variant of claim 1, wherein there is a mutation used to form a disulfide bond between the heavy chain variable region and the light chain variable region, wherein the mutation site comprises any one or more of the following combination forms, according to EU numbering, and the heavy chain variable region is represented by VH, while the light chain variable region is represented by VL:
| | Disulfide bond mutation site | |
|---|---|---|
| | VH | VL |
| Combination 1 | 37C | 95C |
| Combination2 | 44C | 100C |
| Combination 3 | 44C | 105C |
| Combination 4 | 45C | 87C |
| Combination 5 | 100C | 50C |
| Combination 6 | 100bC | 49C |
| Combination 7 | 98C | 46C |
| Combination 8 | 101C | 46C |
| Combination 9 | 105C | 43C |
| Combination 10 | 106C | 57C |

8. The anti-CD3 antibody variant of claim 1, wherein the variant further comprises: a heavy chain constant region selected from human IgG1, IgG2, IgG3, or IgG4 or a variant thereof; and a light chain constant region selected from human κ, λ or a variant thereof; wherein the heavy chain constant region comprises: an Fc fragment or a variant thereof.

9. The anti-CD3 antibody variant of claim 1, wherein the antibody variant is a scFv, which comprises a heavy chain variable region, a light chain variable region, and a linker connecting the heavy chain variable region and the light chain variable region, wherein the amino acid sequence of the linker is preferably several GGGGS replicates, more preferably three GGGGS replicates.

10. Use of the anti-CD3 antibody variant of any one of claims 1-9 in the preparation of drugs for inhibiting or treating cancer.

11. The use of claim 10, wherein the cancer is selected from the following cancers or occurs in the following areas: colorectal, breast, ovary, pancreas, stomach, prostate, kidney, cervix, bone marrow cancer, lymphatic cancer, leukemia, thyroid, endometrium, uterus, bladder, neuroendocrine, head and neck, liver, nasopharyngeal, testicle, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, and myelodysplastic syndrome.

12. A nucleic acid molecule, which encodes the anti-CD3 antibody variant of any one of claims 1-9.

13. An expression vector, which comprises the nucleic acid molecule of claim 12.

14. An anti-tumor associated antigen (TAA)/anti-CD3 protein molecule, wherein the molecule is in a form of heterodimer, comprising a first monomer and a second monomer;
the first monomer comprises: (a) an Fd fragment; (b) a light chain fragment and a first Fc chain;
the second monomer comprises: (a) an Fd fragment; (b) a light chain fragment, an anti-CD3 antibody fragment, and a second Fc chain;
the light chain fragment comprises a VL domain and a CL domain; the Fd fragment comprises a VH domain and a CH1 domain; in the first monomer or the second monomer, the light chain fragment pairs with the Fd fragment to form an anti-TAA Fab domain; wherein the light chain fragment of the first monomer is fused with the first Fc chain; the N-terminus of the anti-CD3 antibody fragment is fused with the light chain fragment of the second monomer, and the C-terminus is fused with the second Fc chain;
or
the first monomer comprises: (a) an Fd fragment; (b) a light chain fragment, a cytokine functional region, and a first Fc chain;
the second monomer comprises: (a) an Fd fragment; (b) a light chain fragment, an anti-CD3 antibody fragment, and a second Fc chain;
the cytokine functional region comprises IL-15 and IL-15Ra; wherein the N-terminus of the cytokine functional region is fused with the light chain fragment of the first monomer, and the C-terminus is fused with the first Fc chain; the N-terminus of the anti-CD3 antibody fragment is fused with the light chain fragment of the second monomer, and the C-terminus is fused with the second Fc chain;
or
the first monomer comprises: an anti-TAA antibody fragment, a cytokine functional region, and a first Fc chain;
the second monomer comprises: an anti-TAA antibody fragment, an anti-CD3 antibody fragment, and a second Fc chain; wherein,
the N-terminus of the cytokine functional region is fused with the anti-TAA antibody fragment of the first monomer, and the C-terminus is fused with the first Fc chain; The N-terminus of the anti-CD3 antibody fragment is fused with the anti-TAA antibody fragment of the second monomer, and the C-terminus is fused with the second Fc chain;
the first Fc chain and the second Fc chain are interchangeable.

15. The anti-tumor associated antigen (TAA)/anti-CD3 protein molecule of claim 14, wherein
the anti-TAA antibody fragment is in the form of scFv, comprising: a heavy chain variable region (VH), a light chain variable region (VL), and a linker connecting the heavy chain variable region and the light chain variable region; from the N-terminus to the C-terminus of the peptide chain, the fusion order of amino acids of the anti-TAA antibody fragment is "VH~VL", or "VL~VH"; wherein "-" represents a linker; or
the anti-CD3 antibody fragment is in the form of scFv, comprising: a heavy chain variable region (VH), a light chain variable region (VL), and a linker connecting the heavy chain variable region and the light chain variable region; from the N-terminus to the C-terminus of the peptide chain, the fusion order of the amino acids of the anti-CD3 antibody fragment is "VH~VL", or "VL~VH"; wherein "-" represents a linker; or
from the N-terminus to the C-terminus of the peptide chain, the fusion order of the amino acid fragments of the cytokine functional region is "IL-15~IL-15Ra", or "IL-15Ra~IL-15"; wherein "-" represents a linker; preferably, the sequence of IL-15~IL-15Ra is shown in SEQ ID NO: 42, and the sequence of IL-15Ra~IL-15 is shown in SEQ ID NO: 43.

16. The anti-tumor associated antigen (TAA)/anti-CD3 protein molecule of claim 14, wherein the amino acid sequence of the anti-CD3 antibody fragment is selected from an antibody, an antibody fragment, a single domain antibody, or a humanized form thereof that specifically binds to CD3; preferably, the amino acid sequence of the anti-CD3 antibody fragment is selected from SP34, OKT3, UCTH1 or a derivative thereof.

17. The anti-tumor associated antigen (TAA)/anti-CD3 protein molecule of claim 14, wherein the anti-CD3 antibody fragment comprises a heavy chain variable region and a light chain variable region; the heavy chain variable region of the anti-CD3 antibody fragment comprises: VHCDR1, VHCDR2, and VHCDR3; the light chain variable region of the anti-CD3 antibody fragment comprises: VLCDR1, VLCDR2, VLCDR3; the amino acid sequences of VHCDR1, VHCDR2, VHCDR3, VLCDR1, VLCDR2, and VLCDR3 are shown in any group of the anti-CD3 antibody variant sequences in claim 5.

18. The anti-tumor associated antigen (TAA)/anti-CD3 protein molecule of claim 14, wherein the IL-15Ra and IL-15 form an IL-15/IL-15Ra complex; IL-15 comprises: IL-15 and mutations, truncations, and various derivants thereof that can bind to IL-15Ra; IL-15Ra comprises: IL-15Ra and mutations, truncations, and various derivatives thereof that can bind to IL-15; preferably, the IL-15/IL-15Ra complex includes but not limited to any one of the mutation modes shown in the following combinations, and the numbering method is to start counting from the first amino acid in the amino acid sequence of IL-15 or IL-15Ra as the first position; preferably, the maternal sequence of IL-15 is shown in SEQ ID NO: 44; the maternal sequence of the IL-15Ra is shown in SEQ ID NO: 45:
| Combination | **IL15** | **IL15Ra** |
|---|---|---|
| 1 | wt | D96 |
| 2 | wt | D96/P97 |
| 3 | wt | D96/P97/A98 |
| 4 | E87C | D96/C97 |
| 5 | E87C | D96/P97/C98 |
| 6 | E87C | D96/C97/A98 |
| 7 | V49C | S40C |
| 8 | L52C | S40C |
| 9 | E89C | K34C |
| 10 | Q48C | G38C |
| 11 | E53C | L42C |
| 12 | C42S | A37C |
| 13 | L45C | G38C |
| 14 | L45C | A37C |

19. The anti-tumor associated antigen (TAA)/anti-CD3 protein molecule of claim 14, wherein the IL-15 includes but is not limited to any one of the mutation modes shown in the following combinations, and the numbering method is to start counting from the first amino acid in the amino acid sequence of the IL-15 as the first position; preferably, the maternal sequence of IL-15 is shown in SEQ ID NO: 44:
| Combination | **IL15 mutation** |
|---|---|
| 1 | N1D |
| 2 | N4D |
| 3 | D8N |
| 4 | D30N |
| 5 | D61N |
| 6 | E64Q |
| 7 | N65D |
| 8 | Q108E |
| 9 | N1D/D61N |
| 10 | N1D/E64Q |
| 11 | N4D/D61N |
| 12 | N4D/E64Q |
| 13 | D8N/D61N |
| 14 | D8N/E64Q |
| 15 | D61N/E64Q |
| 16 | E64Q/Q108E |
| 17 | N1D/N4D/D8N |
| 18 | D61N/E64Q/N65D |
| 19 | N1D/D61N/E64Q/Q108E |
| 20 | N4D/D61N/E64Q/Q108E |

20. The anti-tumor associated antigen (TAA)/anti-CD3 protein molecule of claim 14, wherein the TAA is selected from: CD20, CD19, CD30, CD33, CD38, CD40, CD52, slamf7, GD2, CD24, CD47, CD133, CD239, CD276, PD-1, CEA, Epcam, Trop2, TAG72, MUC1, MUC16, mesothelin, folrl, CLDN18.2, PDL1, EGFR, EGFR VIII, C-MET, HER2, FGFR2, FGFR3, PSMA, PSCA, EphA2, ADAM17, 17-A1, NKG2D ligands, MCSP, LGR5, SSEA3, SLC34A2, BCMA, GPNMB or Glypican-3.

21. The anti-tumor associated antigen (TAA)/anti-CD3 protein molecule of claim 14, wherein the first Fc chain and the second Fc chain are polymerized to form an Fc fragment; the Fc fragment is selected from human IgG1 Fc, human IgG2 Fc, human IgG3 Fc, human IgG4 Fc or a variant thereof, preferably from IgG1 Fc, or human IgG4 Fc or a variant thereof; the protein molecule is in a form of Fc heterodimer; preferably, the Fc heterodimer includes but is not limited to a combination of the following mutations, according to EU numbering:
| Combina tion | FC | Heterodimer mutation (EU numbering) |
|---|---|---|
| 1 | first FC chain | T366Y |
| | second FC chain | Y407T |
| 2 | first FC chain | T366W |
| | second FC chain | T366S/L368A/Y407V |
| 3 | first FC chain | S354C/T366W |
| | second FC chain | Y349C/T366S/L368A/Y407V |
| 4 | first FC chain | S364H/F405A |
| | second FC chain | Y349T/T394F |
| 5 | first FC chain | T350V/L351Y/F405A/Y407V |
| | second FC chain | T350V/T366L/K392L/T394W |
| 6 | first FC chain | K392D/K409D |
| | second FC chain | E356K/D399K |
| 7 | first FC chain | D221EP228E/L368E |
| | second FC chain | D221R/P228R/K409R |
| 8 | first FC chain | K360EK409W |
| | second FC chain | Q347R/D399V/F405T |
| 9 | first FC chain | K360E/K409W/Y349C |
| | second FC chain | Q347R/D399V/F405T/S354C |
| 10 | first FC chain | K370E/K409W |
| | second FC chain | E357ND399VF405T |
| 11 | first FC chain | F405L |
| | second FC chain | K409R |
| 12 | first FC chain | K360D/D399M/Y407A |
| | second FC chain | E345R/Q347R/T366V/K409V |
| 13 | first FC chain | Y349S/K370Y/T366M/K409V |
| | second FC chain | E356G/E357D/S364Q/Y407A |
| 14 | first FC chain | L351D/L368E |
| | second FC chain | L351K/T366K |
| 15 | first FC chain | GQPFRPEVHLIPPSREMTKNQVSLTCLA RGFYPKDAVEWESNGQPENNYKTTPS RQEPSQGTTTFAVTSKLTVDKSRWQQ GNVFSCSVMHEALHNHYTQKTEL |
| | second FC chain | GQPREPQVYTPPPSEEAINELVILTCLV KGFYPSDAVEWLQGSQELPREKYLTW APVLDSDGSFFLYSLRVAAEDWKKGD TFSCSVMHEALHNHYTQKSDR |
| 16 | first FC chain | L368D/K370S |
| | second FC chain | E357Q/S364K |
| 17 | first FC chain | S354C/T366W/**K409A** |
| | second FC chain | Y349C/T366S/L368A/Y407V/**F405K** |
| 18 | first FC chain | S354C/T366W/**F405K/K360E/Q347E** |
| | second FC chain | Y349C/T366S/L368A/Y407V/**Q347R/T394 W** |
| 19 | first FC chain | T366W/**K409A** |
| | second FC chain | T366S/**L368G/Y407A/F405K** |
| 20 | first FC chain | knobs (T366W/**F405K**) |
| | second FC chain | holes (T366S/**L368G/Y407A/K409A**) |
| 21 | first FC chain | Q347A/S364K/T366VK370T/K392YF405S /Y407V/K409W/T411N |
| | second FC chain | Q347E/Y349AL351F/S364T/T366V//K370 T/T394D/V397L/D399E/ D401Q/F405A/Y407S/K409R/T411R |
| 22 | first FC chain | K274Q/N276K/Y300F/A339T/Q347A/S364 |
| | | K/T366V/K370T/**N384S** |
| | | /K392Y/**V397M**/F405S/Y407V/K409W/T4 |
| | | 11N/**V422I/H435R/Y436F** |
| | second FC chain | Q347E/Y349A/L351F/S364T/T366V/K370 |
| | | T/T394D/V397L/D399E/ |
| | | D401Q/F405A/Y407S/K409R/T411R |

22. The anti-tumor associated antigen (TAA)/anti-CD3 protein molecule of claim 14, wherein the protein molecule comprises an Fc fragment that selectively eliminates immune effector functions, including but not limited to a combination of the following mutations, according to EU numbering:
| **IgG** | FC Mutate(EU numbing) |
|---|---|
| | L234A,L235A |
| | L234A,L235A,P329G |
| | L234F,L235E,P331S |
| | D265A,N297A |
| | L234F,L235E,N297A |
| | L234F,L235E,D265A |
| | L234A,L235E,P331S |
| **IgG1** | L234A,L235E,N297A |
| | L234A,L235E,D265A |
| | L234A,L235A,P331S |
| | L234A,L235A,N297A |
| | L234A,L235A,D265A |
| | L235E,D265A,P331S |
| | L235E,N297A,P331S |
| | L235E,N297A |
| | L235A,D265A,P331S |
| | L235A,N297A,P331S |
| | N297Q |
| | N297A |
| | N297G |
| | A287C, N297G, L306C |
| | R292C, N297G, V302C |
| **hIgG4** | S228P,L235E,P329G |
| | S228P,L235E |
| | S228P,F234A,L235E |
| | S228P,F234A,L235A |
| **IgG2m4** | ASTKGPSVFPLAPCSRSTSESTAALGCLVKDYFPEPVTVSWNSGALT SGVHTFPAVLQSSGLYSLSSVVTVPSSNFGTQTYTCNVDHKPSNTKV DKTVERKCCVECPPCPAPPVAGPSVFLFPPKPKDTLMISRTPEVTCVV VDVSQEDPEVQFNWYVDGVEVHNAKTKPREEQFNSTYRVVSVLTV LHQDWLNGKEYKCKVSNKGLPSSIEKTISKAKGQPREPQVYTLPPSQ EEMTKNQVSLTCLVKGFYPSDIAVEWESNGQPENNYKTTPPVLDSD GSFFLYSRLTVDKSRWQEGNVFSCSVMHEALHNHYTQKSLSLSLGK |
| | N297A |
| | N297Q |
| | N297G |

23. The anti-tumor associated antigen (TAA)/anti-CD3 protein molecule of claim 14, which is obtained by fusing amino acid fragments shown in any one of the following groups of sequences:
(1) SEQ ID NO:05, SEQ ID NO:06, SEQ ID NO:07;
(2) SEQ ID NO:08, SEQ ID NO:06, SEQ ID NO:07;
(3) SEQ ID NO:09, SEQ ID NO:06, SEQ ID NO:07;
(4) SEQ ID NO:10, SEQ ID NO:06, SEQ ID NO:07;
(5) SEQ ID NO:05, SEQ ID NO:17, SEQ ID NO:07;
(6) SEQ ID NO:01, SEQ ID NO:18, SEQ ID NO:07;
(7) SEQ ID NO:19, SEQ ID NO:17, SEQ ID NO:07;
(8) SEQ ID NO:19, SEQ ID NO:18, SEQ ID NO:07;
(9) SEQ ID NO:20, SEQ ID NO:21;
(10) SEQ ID NO:22, SEQ ID NO:21;
(11) SEQ ID NO:20, SEQ ID NO:23;
(12) SEQ ID NO:22, SEQ ID NO:23;
(13) SEQ ID NO:24, SEQ ID NO:25;
(14) SEQ ID NO:26, SEQ ID NO:25;
(15) SEQ ID NO:24, SEQ ID NO:27;
(16) SEQ ID NO:26, SEQ ID NO:27.

24. A nucleic acid molecule, which encodes the anti-tumor associated antigen (TAA)/anti-CD3 protein molecule of any one of claims 14-23.

25. Use of the anti-tumor associated antigen (TAA)/anti-CD3 protein molecule of any one of claims 14-23 in the preparation of drugs for inhibiting or treating cancer, wherein the cancer is selected from the following cancers or occurs in the following areas: colorectal, breast, ovary, pancreas, stomach, prostate, kidney, cervix, bone marrow cancer, lymphatic cancer, leukemia, thyroid, endometrium, uterus, bladder, neuroendocrine, head and neck, liver, nasopharyngeal, testicle, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, and myelodysplastic syndrome.

26. An anti-tumor associated antigen (TAA) and IL-15/IL-15Ra-containing fusion protein, which comprises two polypeptide chains, any one of which comprises: an anti-TAA antibody fragment, a cytokine functional region, and an Fc fragment; the anti-TAA antibody fragment is in the form of scFv; the cytokine functional region comprises IL-15 and IL-15Ra; the N-terminus of the cytokine functional region is fused with the anti-TAA antibody fragment, and the C-terminus is fused with the Fc fragment.

27. The anti-tumor associated antigen (TAA) and IL-15/IL-15Ra-containing fusion protein of claim 26, wherein the IL-15Ra and IL-15 form an IL-15/IL-15Ra complex; the IL-15 comprises: IL-15 and mutations, truncations, and various derivatives thereof that can bind to IL-15Ra; the IL-15Ra comprises: IL-15Ra and mutations, truncations, and various derivatives thereof that can bind to IL-15; preferably, the IL-15/IL-15Ra complex includes but is not limited to any one of the mutation modes shown in the following combinations, and the numbering method is to start counting from the first amino acid in the amino acid sequence of IL-15 or IL-15Ra as the first position; preferably, the maternal sequence of IL-15 is shown in SEQ ID NO: 44; the maternal sequence of the IL-15Ra is shown in SEQ ID NO: 45:
| Combination | **IL15** | **IL15Ra** |
|---|---|---|
| 1 | wt | D96 |
| 2 | wt | D96/P97 |
| 3 | wt | D96/P97/A98 |
| 4 | E87C | D96/C97 |
| 5 | E87C | D96/P97/C98 |
| 6 | E87C | D96/C97/A98 |
| 7 | V49C | S40C |
| 8 | L52C | S40C |
| 9 | E89C | K34C |
| 10 | Q48C | G38C |
| 11 | E53C | L42C |
| 12 | C42S | A37C |
| 13 | L45C | G38C |
| 14 | L45C | A37C |

28. The anti-tumor associated antigen (TAA) and IL-15/IL-15Ra-containing fusion protein of claim 26, wherein the IL-15 includes but is not limited to any one of the mutation modes shown in the following combinations, and the numbering method is to start counting from the first amino acid in the amino acid sequence of IL-15 as the first position; preferably, the maternal sequence of IL-15 is shown in SEQ ID NO: 44:
| Combination | **IL15 mutation** |
|---|---|
| 1 | N1D |
| 2 | N4D |
| 3 | D8N |
| 4 | D30N |
| 5 | D61N |
| 6 | E64Q |
| 7 | N65D |
| 8 | Q108E |
| 9 | N1D/D61N |
| 10 | N1D/E64Q |
| 11 | N4D/D61N |
| 12 | N4D/E64Q |
| 13 | D8N/D61N |
| 14 | D8N/E64Q |
| 15 | D61N/E64Q |
| 16 | E64Q/Q108E |
| 17 | N1D/N4D/D8N |
| 18 | D61N/E64Q/N65D |
| 19 | N1D/D61N/E64Q/Q108E |
| 20 | N4D/D61N/E64Q/Q108E |

29. The anti-tumor associated antigen (TAA) and IL-15/IL-15Ra-containing fusion protein of claim 26, wherein the fusion protein comprises an Fc fragment, which selectively eliminates immune effector functions, including but not limited to a combination of the following mutations, according to EU numbering:
| | |
|---|---|
| **IgG** | FC Mutate(EU numbing) |
| **IgG1** | L234A,L235A |
| | L234A,L235A,P329G |
| | L234F,L235E,P331S |
| | D265A,N297A |
| | L234F,L235E,N297A |
| | L234F,L235E,D265A |
| | L234A,L235E,P331S |
| | L234A,L235E,N297A |
| | L234A,L235E,D265A |
| | L234A,L235A,P331S |
| | L234A,L235A,N297A |
| | L234A,L235A,D265A |
| | L235E,D265A,P331S |
| | L235E,N297A,P331S |
| | L235E,N297A |
| | L235A,D265A,P331S |
| | L235A,N297A,P331S |
| | N297Q |
| | N297A |
| | N297G |
| | A287C, N297G, L306C |
| | R292C, N297G, V302C |
| **hIgG4** | S228P,L235E,P329G |
| | S228P,L235E |
| | S228P,F234A,L235E |
| | S228P,F234A,L235A |
| **IgG2m4** | ASTKGPSVFPLAPCSRSTSESTAALGCLVKDYFPEPVTVSWNSGALT SGVHTFPAVLQSSGLYSLSSVVTVPSSNFGTQTYTCNVDHKPSNTKV DKTVERKCCVECPPCPAPPVAGPSVFLFPPKPKDTLMISRTPEVTCVV VDVSQEDPEVQFNWYVDGVEVHNAKTKPREEQFNSTYRVVSVLTV LHQDWLNGKEYKCKVSNKGLPSSIEKTISKAKGQPREPQVYTLPPSQ EEMTKNQVSLTCLVKGFYPSDIAVEWESNGQPENNYKTTPPVLDSD GSFFLYSRLTVDKSRWQEGNVFSCSVMHEALHNHYTQKSLSLSLGK |
| | N297A |
| | N297Q |
| | N297G |

30. The anti-tumor associated antigen (TAA) and IL-15/IL-15Ra-containing fusion protein of claim 26, wherein any polypeptide chain of the fusion protein has an amino acid sequence shown in SEQ ID NO: 28, or has at least 90% sequence identity with it.

31. A nucleic acid molecule, which encodes the anti-tumor associated antigen (TAA) and IL-15/IL-15Ra-containing fusion protein of any one of claims 26-30.

32. Use of the anti-tumor associated antigen (TAA) and IL-15/IL-15Ra-containing fusion protein of any one of claims 26-30 in the preparation of drugs for inhibiting or treating cancer, wherein the cancer is selected from the following cancers or occurs in the following areas: colorectal, breast, ovary, pancreas, stomach, prostate, kidney, cervix, bone marrow cancer, lymphatic cancer, leukemia, thyroid, endometrium, uterus, bladder, neuroendocrine, head and neck, liver, nasopharyngeal, testicle, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, and myelodysplastic syndrome.
